(19) Europäisches Patentamt
European Patent Office
Office européen des brevets

(11) Numéro de publication : **0 237 366**
**B1**

(12) # FASCICULE DE BREVET EUROPÉEN

(45) Date de publication du fascicule du brevet : **26.04.89**

(21) Numéro de dépôt : **87400116.7**

(22) Date de dépôt : **19.01.87**

(51) Int. Cl.[4] : **C 07 C 91/16**, C 07 C103/30, C 07 C103/44, C 07 C103/737, C 07 D295/08, A 61 K 31/135, A 61 K 31/10, A 61 K 31/445// A61K31/495, A61K31/535

(54) **Aminoalcools, leur procédé de préparation et leurs applications, notamment en thérapeutique.**

(30) Priorité : **30.01.86 FR 8601295**

(43) Date de publication de la demande : **16.09.87 Bulletin 87/38**

(45) Mention de la délivrance du brevet : **26.04.89 Bulletin 89/17**

(84) Etats contractants désignés : **AT BE CH DE ES FR GB GR IT LI LU NL SE**

(56) Documents cités :
**FR–A– 2 125 484**
**GB–A– 1 434 826**
**CHEMICAL ABSTRACTS, vol. 87, no. 23, 5 décembre 1977, page 595, no. 184330h, Columbus, Ohio, US; K.M.R. PILLAI et al.: "Agents acting on the central nervous system: part XXVII - synthesis of 2-methyl-2-(1'-piperidyl)-3-phenylpropanol and some related compounds"**
**Neurochemistry (F.Hucho, 1986, pp. 157-249)**
**Use of Automated Tail Suspension Test for the Primary Screening of Psychotropic Agents, Arch. int. Pharmacodyn 288 p.11-30, 1987**
**Psychotoniques et Antidépresseurs,(Vol.III. p.267-321, 1970. Masson ed.**
**Le dossier contient des informations techniques présentées postérieurement au dépôt de la demande et ne figurant pas dans le présent fascicule.**

(73) Titulaire : **JOUVEINAL S.A.**
**Tour Maine Montparnasse 33 Avenue du Maine**
**F-75755 Paris Cedex 15 (FR)**

(72) Inventeur : **Aubard, Gilbert**
**7 Chemin de la Savetière**
**F-91120 Palaiseau (FR)**
Inventeur : **Bure, Jacques**
**22, rue Perronet**
**F-92200 Neuilly-sur-Seine (FR)**
Inventeur : **Calvet, Alain Pierre**
**10, rue Madame**
**F-78000 Versailles (FR)**
Inventeur : **Gouret, Claude**
**34, rue Croix du Val**
**F-92190 Meudon (FR)**
Inventeur : **Grouhel, Agnès Geneviève**
**2, rue des Peupliers**
**F-92190 Meudon (FR)**
Inventeur : **Junien, Jean-Louis**
**85 Boulevard Suchet**
**F-75016 Paris (FR)**

(74) Mandataire : **Bourgognon, Jean-Marie et al**
**Cabinet Flechner 22, Avenue de Friedland**
**F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**EP 0 237 366 B1**

Jouve, 18, rue St-Denis; 75001 Paris, France

**Description**

La présente invention concerne de nouveaux aminoalcools dérivés du 2-(3, 4-dichlorobenzyl)-2-aminoéthanol, leur procédé de préparation et leurs applications, notamment en thérapeutique.

Aux lignes 37 à 42 de la page 2 du brevet Gallardo, on a tracé une formule très générale d'alcools qui englobe les composés suivant l'invention, mais on n'en donne aucun exemple. Ces composés ne sont pas présentés comme ayant une activité pharmacologique, mais comme intermédiaires qui servent à préparer des esters qui n'ont pas d'activité sur le système nerveux central.

Au FR-A-2 125 484, on décrit des composés dont l'atome de carbone en position α de l'atome d'azote n'est pas tetrasubstitué. Ces composés n'ont pas d'affinité pour les sites de recapture de la sérotonine. De même, les composés monochlorés décrit aux « CHEMICAL ABSTRACTS », vol. 87, n° 23, 5 décembre 1977, page 595, n° 184330h, Columbus, Ohio, US ; K.M.R. PILLAI et al. : « Agents acting on the central nervous system : part XXVIII » n'ont pas non plus de propriété sérotoninergique.

L'invention a pour objet de nouveaux aminoalcools de formule

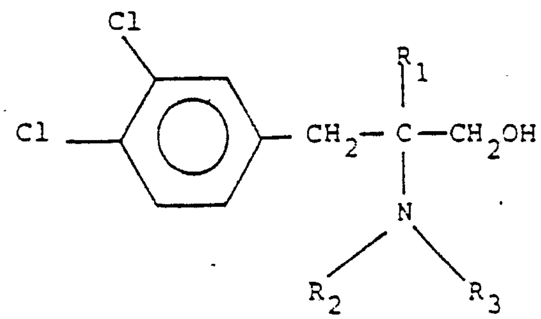

(I)

dans laquelle :

$R_1$ est alkyle inférieur,

$R_2$ est H ou alkyle inférieur,

$R_3$ est H, alkyle inférieur, alkényle inférieur, phénylalkyle inférieur ou cycloalkylalkyle inférieur ayant de 3 à 6 atomes de carbone dans le cycle, ou, quand $R_2$ est H, alkanoyle inférieur, phénylalkanoyle inférieur ou cycloalkylcarbonyle ayant de 3 à 6 atomes de carbone dans le cycle, ou $R_2$ et $R_3$ forment, ensemble avec l'atome d'azote auquel ils sont reliés, un hétérocycle saturé de cinq à sept chaînons pouvant comprendre, comme second hétéroatome non relié directement à l'atome d'azote, un oxygène, un soufre ou un azote, ce dernier hétéroatome d'azote pouvant porter un substituant alkyle en $C_1$ à $C_4$, et leurs sels d'addition avec des acides.

Les radicaux alkyle et alkanoyle inférieur sont linéaires ou ramifiés et comprennent de 1 à 5 atomes de carbone ; le radical alkényle a, de préférence, de 2 à 5 atomes de carbone et, notamment, 3 atomes de carbone, tandis que les radicaux cycloalkyle ont, de préférence, de 3 à 6 atomes de carbone.

Parmi les acides, notamment ceux thérapeutiquement acceptables, qui forment des sels d'addition avec les composés suivant l'invention, on peut citer à titre d'exemple des acides minéraux ou organiques, comme les acides acétique, benzènesulfonique, camphosulfonique, citrique, éthanesulfonique, fumarique, bromhydrique, chlorhydrique, lactique, maléique, malique, méthanesulfonique, nitrique, pamoïque, phosphorique, salicyclique, stéarique, succinique, sulfurique, tartrique.

L'invention concerne tant les formes racémiques que les formes optiquement actives des composés de formule (I) précisément définis.

Les composés suivant l'invention présentent des activités sur le système nerveux central (SNC) ainsi que des propriétés analgésiques qui sont inattendues dans des aminoalcools. L'invention vise donc l'utilisation des composés (I) à titre de médicament analgésique et agissant sur le système nerveux central.

Parmi les composés de l'invention, on préfère ceux dans lesquels $R_1$ est méthyle, $R_2$ est hydrogène ou méthyle, et $R_3$ est hydrogène ou méthyle.

L'invention vise également un procédé de préparation des composés de l'invention qui consiste :

à réduire un acide de formule

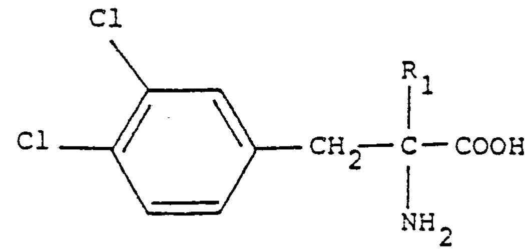

(II)

par un hydrure de bore ou d'aluminium pour obtenir les composés (Ia) dans lesquels $R_2$ et $R_3$ sont l'hydrogène, et

pour obtenir les composés (Ib), dans lesquels $NR_2R_3$ forment un hétérocycle, à effectuer une cyclisation par réaction d'un composé (Ia) sur un réactif de formule

$$X—(CH_2)_m—R_5—(CH_2)_n—X'$$

dans laquelle X et X', semblables ou différents, sont halogènes, $R_5$ est une liaison simple de valence et m et n sont des nombres entiers allant de 1 à 3, avec m + n supérieur ou égal à 4 et inférieur ou égal à 6, ou $R_5$ est un oxygène, un soufre ou $NR_6$, $R_6$ étant H ou alkyle en $C_1$ à $C_4$ et m et n sont des nombres entiers allant de 1 à 3 avec m + n supérieur ou égal à 3 et inférieur ou égal à 5, et

pour préparer les composés (Ic) dans lesquels $R_3$ est alkanoyle, phénylalkanoyle ou cycloalkylcarbonyle, à acyler un composé (Ia) par un agent d'acylation de formule $R_4$-COX dans lequel X est un halogène et $R_4$ est l'homologue immédiatement inférieur de $R_3$, et

pour préparer les composés Id, dans lesquels $R_2$ est H et $R_3$ est alkyle, alkényle, phénylalkyle, ou cycloalkylalkyle, à alkyler un composé (Ia) par un agent d'alkylation $R_3$-X, X étant un halogène, ou à réduire un composé (Ic) par un hydrure de bore ou d'aluminium, ou à réduire un composé de formule

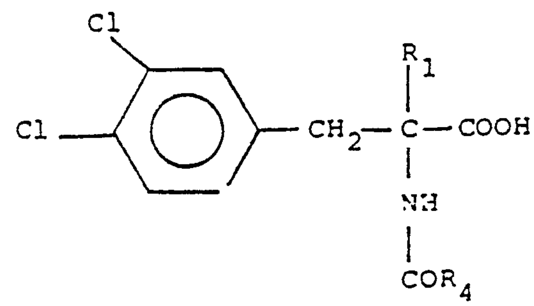

(IV)

par un hydrure de bore ou d'aluminium, et

pour préparer les composés (Ie), dans lesquels $R_2$ est alkyle et $R_3$ est identique à $R_2$, à faire réagir un composé (Ia) sur un aldéhyde de formule $R_4CHO$ en présence d'acide formique, et

pour préparer les composés (Ie) dans lesquels $R_2$ est alkyle et $R_3$ est alkyle, alkényle, phénylalkyle ou cycloalkylalkyle, à faire réagir un composé (Id) sur un aldéhyde de formule $R_4CHO$ en présence d'acide formique et, pour préparer les sels d'addition avec des acides, à faire réagir les composés (I) sur un acide.

Le procédé de préparation des composés suivant l'invention est résumé au tableau A ci-après, le radical dichlorobenzyle n'étant pas représenté dans les formules des composés du tableau A.

(Voir Tableau A page 4)

Les produits de départ pour la préparation des composés suivant l'invention sont des aminoacides racémiques ou optiquement actifs de formules (II) et (IV)

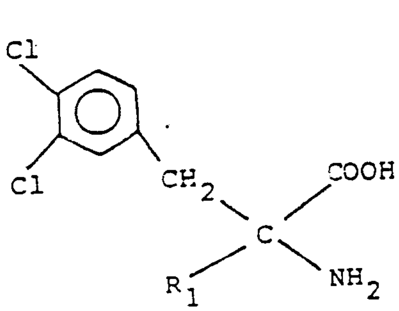

(II)

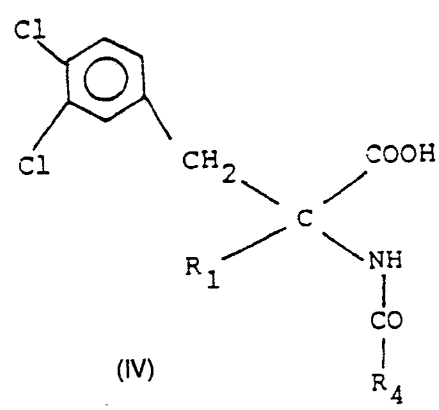

(IV)

dans lesquelles $R_1$ est un radical alkyle inférieur de définition identique à celle de la formule I, $R_4$ est l'hydrogène, un radical alkyle inférieur, un radical phénylalkyle inférieur ou encore un radical cycloalkyle, $R_4$ étant l'homologue immédiatement inférieur de $R_3$ ($R_3$ = $—CH_2R_4$).

Les composés de formules II et IV peuvent être préparés par divers procédés, dont celui décrit aux exemples 1 et 2 du brevet français No. 77 02360, qui consiste dans un premier temps à condenser le

Tableau A

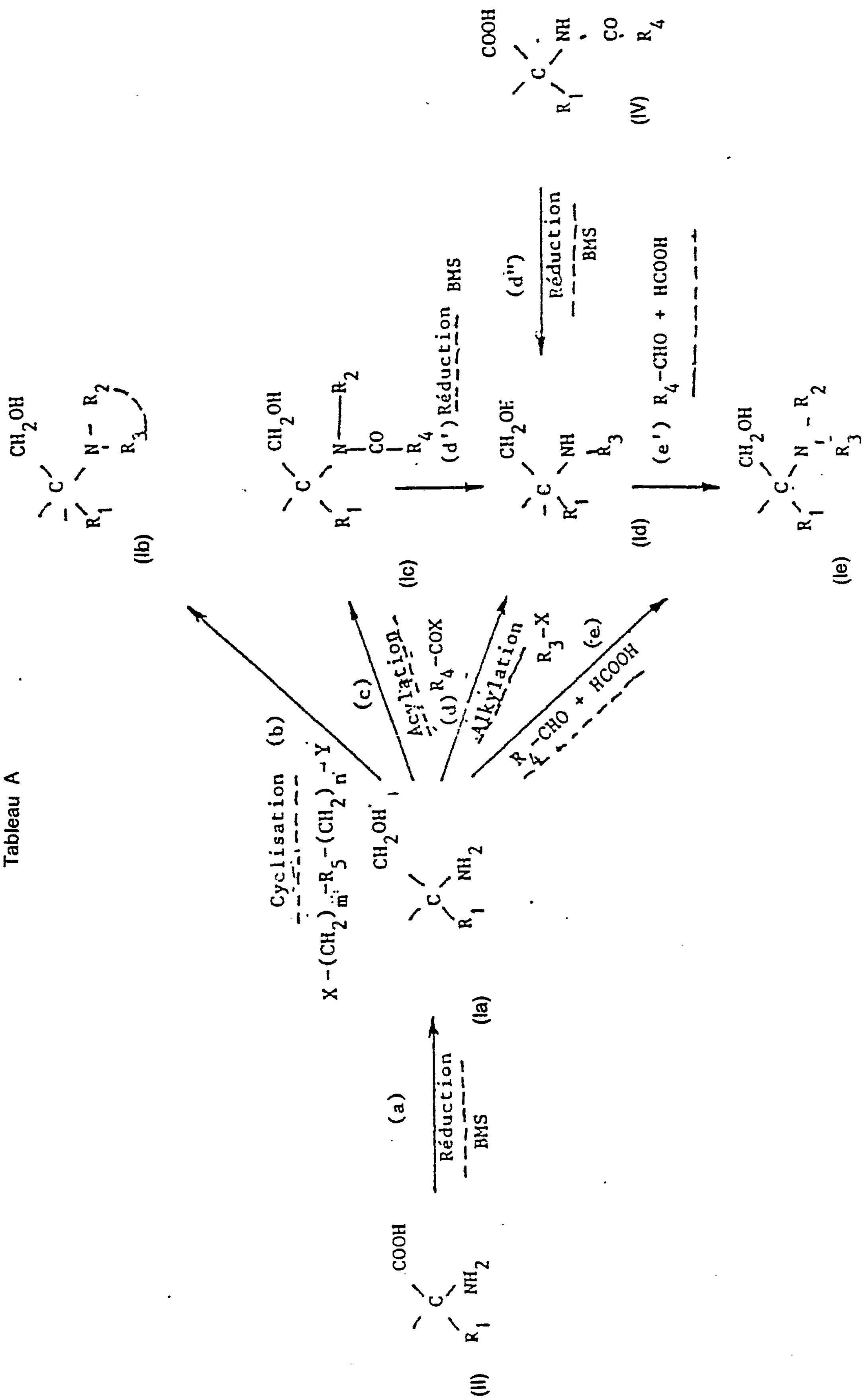
(II)
(a)
Réduction
BMS
(Ia)
Cyclisation
X -(CH2)m-R5-(CH2)n-Y
(b)
(Ib)
(c)
Acylation
R4-COX
(Ic)
(d)
Alkylation
R3-X
(e)
R4-CHO + HCOOH
(d') Réduction BMS
(Id)
(d'')
Réduction
BMS
(IV)
(e') R4-CHO + HCOOH
(Ie)

chlorure ou le bromure de 3,4-dichlorobenzyle avec des isocyano-esters de formule III

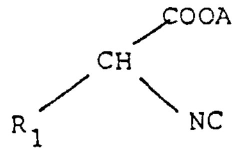

(III)

dans lesquels $R_1$ est un radical alkyle inférieur tel que précédemment défini et A un radical alkyle de faible masse moléculaire pour obtenir, selon l'exemple 1 du brevet, des aminoacides racémiques de formule II.

Appliqué à ces composés, le procédé de l'exemple 2 de ce même brevet permet d'obtenir d'une part les produits de formule IV sous formes racémiques et optiquement actives et d'autre part les aminoacides de formule II sous formes optiquement actives.

Comme le montre le tableau A, les composés Ia, Ib, Ic, Id et Ie dans lesquels $R_1$ est un radical alkyle inférieur précédemment défini, répondent aux composés de l'invention de formule générale I et sont préparés à partir des composés II et IV par les réactions suivantes :

les composés de formule Ia sont obtenus par réduction des fonctions carboxyliques des composés II sous l'action d'agents réducteurs comme les hydrures de bore ou d'aluminium selon la réaction (a),

les composés de formule Ib sont obtenus par réaction de cyclisation sur la fonction amine des composés Ia selon la réaction (b) avec des composés de formule :

$$X—(CH_2)_m—R_5—(CH_2)_n—X'$$

dans lesquels X et X' semblables ou différents sont des atomes d'halogène comme l'iode, le brome ou le chlore, $R_5$ est une liaison de valence ou représente un atome d'oxygène, de soufre, ou $NR_6$, $R_6$ étant H ou alkyle en $C_1$ à $C_4$, m et n ont pour valeur 1, 2 ou 3. Les composés Ib obtenus sont caractérisés en ce que les radicaux $R_2$ et $R_3$ de la formule I forment, avec l'atome d'azote auxquels ils sont reliés, un hétérocycle saturé de 5 à 7 atomes qui peut comprendre un second hétéroatome qui est l'azote, l'oxygène ou le soufre,

les composés de formule IC sont préparés selon la réaction (c) par N-acylation des composés Ia avec des halogénures d'alkanoyle de formule

$$R_4—COX$$

dans lesquels X est un atome d'halogène comme le chlore ou le brome, $R_4$ est un radical alkyle inférieur, ou phénylalkyle inférieur, $R_4$ étant le radical immédiatement inférieur de $R_3$ ($R_3 = CH_2R_4$), ou bien $R_4$ est cycloalkyle.

Les composés de structures Id sont préparés à partir des composés Ia, Ic, IV au moyen des réactions suivantes :

(d) la réaction des halogénures d'alkényle de formule :

$$R_3—X$$

dans lesquels X est un atome d'halogène comme le brome et $R_3$ un alkényle comme le radical allyle avec les composés de formule Ia conduit aux dérivés Id correspondants,

(d') consiste en la réduction de la fonction N-alkoyle des composés Ic précédemment décrits par des agents réducteurs identiques à ceux de la réaction (a) pour obtenir les composés Id dans lesquels $R_3$ est un radical alkyle inférieur, un radical phénylalkyle inférieur, un radical cycloalkylalkyle,

(d") consiste à réduire à la fois les fonctions carboxylique et N-alkoyle des composés IV précédemment décrits par les agents réducteurs déjà cités afin d'obtenir les composés Id identiques à ceux obtenus par la réaction (d'),

la préparation des composés de structure Ie est effectuée à partir des composés Ia, et Id selon les réactions qui suivent :

(e) les aminoalcools de formule Ia par dialkylation réductrice sous l'action d'aldéhydes de formule $R_4—CHO$, dans lesquels $R_4$ est l'hydrogène ou un radical alkyle inférieur, et d'acide formique que permettent d'obtenir les composés de structure I dans lesquels $R_2$ et $R_3$ identiques sont des radicaux alkyle inférieur et plus particulièrement des radicaux méthyle,

(e') par monoalkylation réductrice comme décrit dans la réaction (e), les composés de structure Id permettent d'obtenir les composés de l'invention Ie dans lesquels $R_2$ est un radical alkyle inférieur et plus particulièrement méthyle, $R_3$ étant les radicaux correspondants de Id obtenus par les réactions (d) (d') et (d").

Ainsi qu'il vient d'être décrit, les produits de l'invention sont obtenus par les réactions qui suivent :

— réductions (a), (d'), (d") et

— mono ou dialkylation (b), (d), (e) et (e')

— acylation (c)

Les réactions de réduction consistent à réduire les groupements carbonyle des fonctions carboxyliques et/ou les fonctions amides pour obtenir respectivement des fonctions alcool primaire et/ou des fonctions amine secondaire.

Ces réductions font appel à des méthodes et des réactifs connus qui sont résumés dans le tableau récapitulatif présenté dans « Advanced Organic Chemistry » p. 1118 (J. March $2^e$ éd. française 1977 — McGraw Hill). Les agents réducteurs comme les dérivés de l'hydrure de bore et de l'hydrure d'aluminium sont indiqués comme efficaces pour effectuer ces réactions, en particulier l'hydrure de lithium-aluminium et le complexe borane-diméthylsulfure (BSM dans la suite du texte) est le réactif préféré.

Les réductions s'effectuent en milieux aprotiques non dissociants dans des solvants tels que l'éther diéthylique, l'éther diisopropylique, le 1,2-diméthoxyéthane ou encore le tétrahydrofuranne (THF) qui est le solvant préféré.

Le complexe BMS est utilisé en excès stoechiométrique par rapport aux produits à réduire afin d'obtenir une réaction complète. Cet excès est différent selon la nature des structures mises en jeu et dépend d'une part du nombre de groupements carbonyles à réduire et d'autre part de la présence dans ces structures d'autres groupements ou fonctions réagissant avec le BMS. Ainsi, selon les cas, il est utilisé de 2 à 8 moles de BMS par mole de produit à traiter, les quantités préférées étant toutefois de 2,5 à 3,5 moles de BMS par mole de composé Ic (réaction d') et de 4 à 6 moles par mole de composés II et IV (réactions a et d").

Les produits soumis aux réactions de réductions, à savoir 1 à 10 parties en poids sont introduits à l'abri de l'humidité et sous atmosphère d'azote dans 100 parties en volume de THF et plus particulièrement de 3 à 7 parties dans 100. La quantité de BMS précédemment définie selon les cas, est introduite puis le mélange est chauffé au reflux pour avoir une réaction totale, durée qui peut être comprise entre 30 minutes et 10 heures, cependant une durée de 3 à 5 heures est la plus favorable. L'aminoalcool est alors libéré de son complexe avec l'agent réducteur par addition de méthanol puis d'hydroxyde de sodium et enfin isolé par des procédés conventionnels décrits dans les exemples présentés.

Les réactions de mono et de dialkylation sont réalisées soit par action d'un halogénure d'alkyle soit par action d'un aldéhyde et d'acide formique nommée alkylation réductrice.

Dans le premier cas (réactions b et d) les solvants utilisés sont inertes vis-à-vis des halogénures d'alkyle comme par exemple le toluène et l'acétonitrile. D'une façon générale, pour une mole de produit à alkyler on utilise de 0,5 à 1,5 mole d'halogénure en solution dans 2 à 3 litres du solvant choisi. Le rapport préféré est de 0,85 à 1,20 mole de bromure ou d'iodure d'alkyle par mole de produit mis en réaction. Optionnellement il est ajouté une base qui peut être minérale, comme le carbonate de sodium anhydre, ou organique, comme la triéthylamine, pour favoriser la réaction. Les conditions de température et de temps de la réaction sont adaptées à la nature du solvant et à la réactivité des produits et peuvent être comprises entre 20 et 100 °C durant 1 à 24 heures. Les températures et durées convenables sont de 40 à 110 °C durant 2 à 5 heures, les produits alkylés étant ensuite séparés et purifiés par des méthodes habituelles décrites dans les exemples.

Dans le second cas d'alkylations réductrices (réactions e et e'), les réactions consistent à faire réagir dans un premier temps les aldéhydes $R_4$—CHO précédemment définis avec le produit à alkyler, puis à soumettre les composés intermédiaires non isolés à une réduction par l'acide formique pur, la première partie de la réaction étant effectuée à une température comprise entre 20 et 50 °C, la seconde entre 60 et 100 °C et ce durant une à cinq heures.

D'une façon générale les mono ou dialkylations effectuées par cette méthode nécessitent pour une mole de produit à alkyler de 1,2 à 3,5 moles d'aldéhyde pur ou en solution aqueuse à 30-40 % en poids pour le formaldéhyde et de 2 à 5 moles d'acide formique pur, l'action réductrice de l'acide formique étant réalisée par chauffage du mélange durant 1 à 2 heures à 90-100 °C.

Les réactions d'acylation (c) précédemment définies sont réalisées soit en milieu monophasique anhydre comme le toluène et en présence d'une base organique comme la triéthylamine, soit en milieu biphasique comme dans un mélange eau-1,2-dichloroéthane en présence d'hydroxyde de sodium. Dans les deux cas, le chlorure d'acide est utilisé en rapport stoechiométrique ou en défaut par rapport à l'aminoalcool engagé.

Généralement, pour une molécule d'aminoalcool dans 2 à 10 l de solvant, la base minérale ou organique est introduite à froid, puis le chlorure d'acide à raison de 0,5 à 1 mole. La réaction est conduite en agitant le mélange durant 1 à 5 heures à une température comprise entre 0 et 30 °C et de préférence de 1 à 2 heures, à une température comprise entre 10 et 20 °C, avant de le traiter pour isoler le produit.

Aux lignes 37 à 42 de la page 2 du brevet britannique 1.434.826, on a tracé sur le papier une formule très générale d'alcools qui englobe les composés suivant l'invention. On n'en donne pas un procédé de préparation détaillé, ni aucun exemple précis. Ces alcools ne sont pas présentés comme ayant une activité pharmacologique. Il s'agit en réalité de composés nouveaux, qui n'ont jamais été préparés et la formule jetée sur le papier n'est qu'une simple conjecture.

L'invention est illustrée de façon non limitative par les exemples qui suivent dans lesquels les techniques utilisées sont habituelles à l'homme de l'art. Il est cependant nécessaire de préciser certaines pratiques non détaillées dans les exemples.

— Lors des procédés d'extraction, les phases organiques qui contiennent les produits attendus sont lavées par extractions à l'eau ou avec une solution saturée en chlorure de sodium jusqu'à neutralité de

ces lavages. Leur eau résiduelle est éliminée avant évaporation du solvant par un séjour de quelques heures sur un agent déshydratant, comme le sulfate de sodium anhydre.

— les évaporations de solvants sont effectuées sous vide et sur un bain-marie dont la température est adaptée au point d'ébullition du solvant à éliminer,

— dans le cas des produits purifiés par cristallisation, les solvants utilisés à ces fins sont cités dans le texte ou à la suite de la vapeur du point de fusion du produit obtenu,

— les produits isolés sous forme solide, cristalline ou amorphe sont débarrassés de leurs solvants résiduels par chauffage sous vide jusqu'à poids constant.

— les chlorhydrates des produits sont systématiquement préparés selon le mode opératoire décrit à l'exemple 1.

Par ailleurs, la pureté, l'identité et les caractéristiques physico-chimiques des produits de l'invention sont déterminées par des méthodes rappelées brièvement :

Les points de fusion sont déterminés par la méthode au tube capillaire avec un appareil « Mettler FPI » et ne sont pas corrigés,

la pureté est vérifiée par chromatographie sur couches minces,
support : gel de silice 60 F 254 (fournisseur Merck),
dépôts : 100 mcg environ,
élution : ascendante sur 10 cm dans les mélanges
A — butanol-acide acétique-eau 8-2-2 (v/v/v)
B — chlorure de méthylène-méthanol 9-1 (v/v)
C — chlorure de méthylène-méthanol 9,5-0,5 (v/v)
D — chlorure de méthylène-acétone 8-2 (v/v)
révélation : observation des plaques sous lumière ultraviolette de 254 nm de longueur d'onde, puis observation de la plaque après pulvérisation du réactif de Dragendorff.

Dans les exemples, les solvants de migration utilisés ainsi que les Rf observés sont reportés.

La détermination des pouvoirs rotatoires est effectuée en tubes de 2 dm de longueur.

L'identité des produits obtenus avec les structures proposées est vérifiée par leurs spectres de résonance magnétique nucléaire du proton ($^1$H RMN) à 60 MHz ; les produits sont dissous dans le deutérochloroforme ($CDCl_3$) seul ou en mélange avec le diméthyle sulfoxyde (D-6).

La nature des signaux, leurs déplacements chimiques exprimés en p.p.m. par rapport au tétraméthyle silane pris comme référence, ainsi que le nombre de protons qu'ils représentent sont notés pour chaque spectre. Les protons dits « échangeables » après addition d'oxyde de deutérium sont également signalés.

Dans le texte des abréviations courantes sont utilisées telles que THF pour tétrahydrofurane, BMS pour borane-diméthyle sulfure.

## Exemple 1

1A — (±) 2-méthyl-amino-3-(3,4-dichlorophényl)propanol (JO 1275)

Formule I = $R_1$ = $CH_3$ ; $R_2$ = $R_3$ = H.

Dans un réacteur protégé de l'humidité et sous atmosphère d'azote, 625 ml de tétrahydrofurane (THF) sont introduits, puis 24,8 g (0,1 mol) de (±) 2-(3,4-dichlorobenzyl)alanine.

Dans la suspension obtenue, en 30 minutes et à une température de 20 °C, 38,0 g (0,5 mol) de complexe borane-diméthyl sulfure (BMS) sont ajoutés goutte à goutte. L'agitation est maintenue 15 minutes à température ambiante, puis le mélange est chauffé 4 heures 30 au reflux. Après refroidissement à 5 °C, 75 ml de méthanol sont introduits peu à peu sans dépasser 20 °C, puis d'une façon identique 75 ml de solution d'hydroxyde de sodium N. La suspension obtenue est abandonnée une nuit, l'insoluble est filtré et éliminé. Le filtrat évaporé sous vide et sur bain-marie donne un résidu blanc qui est repris par 500 ml d'eau. Le mélange est acidifié jusqu'à pH 1 par addition d'acide chlorhydrique concentré (d = 1,18).

La solution obtenue est extraite par deux fois 150 ml d'éther. Les phases éthérées sont écartées et la phase acide est alcalinisée à froid jusqu'à pH 12 par addition d'une solution d'hydroxyde de sodium concentrée (d = 1,38) puis saturée en chlorure de sodium.

Le mélange alcalin est extrait par 3 fois 200 ml d'éther, les phases éthérées réunies lavées par une solution saturée en chlorure de sodium, puis séchées.

Après évaporation de l'éther, l'aminoalcool est obtenu sous forme d'un résidu solide, blanc et amorphe.

Poids : 21,5 g — Rendement = 91,8 %
F = 96 °C — CCM : A ; 0,7.

$^1$H-RMN ($CDCl_3$) : 0,95 (s, 3H) ; 2,25 (s, 3H échang.) ; 2,65 (s, 2H) ; 3,30 (s, 2H) ; 6,90-7,40 (m, 3H).

— Chlorhydrate — 21 g (0,09 mol) d'aminoalcool sont dissous dans 220 ml de chlorure de méthylène. Sous agitation et sans dépasser 10 °C, 90 ml d'éther chlorhydrique 3N sont introduits. Après 15 minutes

les solvants sont évaporés et le résidu est à nouveau traité de façon identique avec 220 ml de chlorure de méthylène et 60 ml d'éther chlorhydrique 3N. Le produit brut obtenu après évaporation est purifié par cristallisation dans un mélange de 150 ml d'éthanol et de 300 ml d'éther. Le produit purifié, cristallisé est filtré et séché sous vide à 40 °C.

Poids = 19,6 g Rendement = 80,5 %
F = 171 °C.

Analyse $C_{10}H_{14}Cl_3NO$

| | | | | | |
|---|---|---|---|---|---|
| Calculée : | C 44,39 | H 5,22 | Cl 39,31 | N 5,18 | O 5,91 % |
| Trouvée : | C 44,44 | H 5,15 | Cl 39,20 | N 5,16 | O 6,05 % |

1B — (+) 2-méthyl-2-amino-3-(3,4-dichlorophényl)propanol (JO 1307)

Formule I : $R_1 = CH_3$ ; $R_2 = R_3 = H$.

La réduction selon le mode opératoire précédent de 23,0 g (92 mmol) de (+) 2-(3,4-dichlorobenzyl)alanine permet d'obtenir l'aminoalcool dextrogyre correspondant.

Poids : 20,0 g Rendement = 92,8 %
F = 86 °C CCM : A ; 0,7.

$[\alpha]_D^{20} = + 1,6°$ (C = 5 %, méthanol).
$^1$H-RMN ($CDCl_3$) identique au produit de l'exemple 1A.

Chlorhydrate — Rendement = 92,2 % F = 144 °C (acétone).
Analyse $C_{10}H_{14}Cl_3NO$

| | | | | | |
|---|---|---|---|---|---|
| Calculée : | C 44,39 | H 5,22 | Cl 39,31 | N 5,18 | O 5,91 % |
| Trouvée : | C 44,33 | H 5,24 | Cl 39,20 | N 5,10 | O 5,75 % |

1C — (—) 2-méthyl-2-amino-3-(3,4-dichlorophényl)propanol

Formule I : $R_1 = CH_3$ ; $R_2 = R_3 = H$.

Par un mode opératoire identique aux exemples précédents, l'aminoalcool lévogyre est obtenu par réduction de 60,0 g (242 mmol) de (—) 2-(3,4-dichlorobenzyl)alanine. Le produit brut est purifié par cristallisation dans un mélange éther-hexane.

Poids : 55,6 g Rendement = 98,1 %
F = 85 °C CCM : A ; 0,7

$[\alpha]_D^{20} = — 1,8°$ (C = 5 %, méthanol)
$^1$H-RMN ($CDCl_3$) identique à 1A et 1B.

## Exemple 2

(±) 2-isopropyl-2-amino-3(3,4-dichlorophenyl)propanol (JO 1579)

Formule I — $R_1 = CH(CH_3)_2$ ; $R_2 = R_3 = H$.

Le produit est préparé selon le mode opératoire de l'exemple 1A à partir de 67,2 g (0,43 mol) de (±) 2-isopropyl-2-(3,4 dichlorobenzyl)glycine.

Après évaporation du solvant d'extraction, le produit est obtenu sous forme d'un solide blanc amorphe.

Poids : 39,0 g Rendement = 61,2 %
F = 85 °C CCM : A ; 0,7.

$^1$H-RMN ($CDCl_3$) : 0,85 (d, 3H) ; 1,00 (d, 3H ; 1,35-2,00 (m, 4H, 3H échang.) ; 2,65 (s, 2H) ; 6,90-7,45 (m, 3H).

Chlorhydrate — Rendement = 83,5 % — F = 176,5 °C (acétate d'éthyle)
Analyse $C_{12}H_{18}Cl_3NO$, 0,75 $H_2O$

| | | | | | |
|---|---|---|---|---|---|
| Calculée : | C 46,17 | H 6,25 | Cl 34,08 | N 4,48 | O 8,96 % |
| Trouvée : | C 46,16 | H 6,15 | Cl 34,34 | N 4,43 | O 8,84 % |

## Exemple 3

(±) 2-pentyl-2-amino-3(3,4-dichlorophenyl)propanol (JO 1562)

Formule I — $R_1$ = $(CH_2)_4$—$CH_3$ ; $R_2$ = $R_3$ = H.

Le produit est préparé selon le mode opératoire de l'exemple 1A à partir de 60,0 g (0,197 mol) de (±) 2-pentyl-2-(3,4 dichlorobenzyl)glycine.

Poids : 43,0 g — Rendement = 75,2 %
F = 36 °C (hexane) — CCM : A ; 0,85.

$^1$RMN ($CDCl_3$) : 0,95 (t, 3H) ; 1,35 (s, 8H) ; 2,90 (m, 3H, échang.) ; 2,75 (s, 2H) ; 3,35 (s, 2H) ; 7,00-7,50 (m, 3H).

Chlorhydrate — Rendement = 82,1 % — F = 155 °C (acétone)
Analyse $C_{14}H_{22}Cl_3NO$

| | C | H | Cl | N | O |
|---|---|---|---|---|---|
| Calculée : | C 51,47 | H 6,79 | Cl 32,56 | N 4,28 | O 4,90 % |
| Trouvée : | C 51,33 | H 6,73 | Cl 32,42 | N 4,20 | O 4,89 % |

## Exemple 4

4A — Stade 1 : (±) N-formyl-2-(3,4-dichlorobenzyl)alanine

Intermédiaire IV $R_1$ = $CH_3$ ; $R_4$ = H.

Dans un appareillage protégé de l'humidité, 49,6 g (0,2 mol) de (±) 2-(3,4-dichlorobenzyl)alanine sont dissous dans 80,0 ml d'acide formique pur (d = 1,22). Sous agitation, le mélange est chauffé à 60 °C et la solution ainsi obtenue est maintenue 15 minutes à cette température puis 63,0 ml d'anhydride acétique pur sont introduits en 40 minutes et à une température comprise entre 58 et 62 °C.

Après 15 minutes, 13,0 ml d'eau sont introduits lentement et la solution est progressivement refroidie. Vers 40 °C la cristallisation s'amorce, elle est complétée sous agitation à 5 °C durant 2 heures.

Le produit est filtré, lavé à l'eau glacée jusqu'à neutralité des eaux de lavage, puis séché à 80 °C sous vide jusqu'à poids constant.

Poids : 32,6 g — Rendement = 59,0 %.

$^1$H-RMN (DMSO-$CDCl_3$) 1,45 (s, 3H) ; 3,25 (s, 2H) ; 6,95-7,50 (m, 3H) ; 7,90 (s, 1H échang.) ; 8,00 (d, 1H) ; 10,65 (s large, 1H échang.).

Stade 2 : (±) 2-méthyl-2-méthylamino-3-(3,4-dichlorophényl)propanol (JO 1276)

Formule I : $R_1$ = $R_2$ = $CH_3$ ; $R_3$ = H.

Sous atmosphère d'azote et à l'abri de l'humidité, 21,3 g (77,1 mmol) du produit obtenu au stade 1 précédent sont dissous dans 400 ml de THF.

A 20 °C et en 30 minutes, 29,2 g (385 mmol) de complexe BMS sont introduits. Le mélange est ensuite chauffé durant 4 heures 30 au reflux puis refroidi vers 5 °C.

Successivement et goutte à goutte 65,0 ml de méthanol, puis 65 ml de solution d'hydroxyde de sodium sont introduits sans dépasser 20 °C.

L'insoluble est filtré et éliminé, le résidu obtenu après concentration du filtrat est dissous dans 350 ml d'acide chlorhydrique normal.

La solution est extraite 3 fois par 125 ml d'éther puis alcalinisée à pH 12 par une solution de soude concentrée et saturée en chlorure de sodium.

Le mélange alcalin est extrait 3 fois par 200 ml de chlorure de méthylène. Les phases organiques réunies sont traitées de façon habituelle et après évaporation le produit est obtenu sous forme d'un solide blanc, amorphe.

Poids : 13,4 g — Rendement = 70,1 %
F = 123 °C — CCM : A ; 0,5.

$^1$H-RMN (DMSO-$CDCl_3$) 0,95 (s, 3H) ; 2,35 (s, 4H dont 1H échang.) ; 2,65 (s, 3H dont 1H échang.) ; 3,25 (s, 2H) ; 6,95-7,50 (m, 3H).

Chlorhydrate — Rendement = 73,0 % F 226°C (éthanol).
Analyse $C_{11}H_{16}Cl_3NO$

| | C | H | Cl | N | O |
|---|---|---|---|---|---|
| Calculée : | C 46,42 | H 5,67 | Cl 37,37 | N 4,92 | O 5,62 % |
| Trouvée : | C 46,58 | H 5,67 | Cl 37,30 | N 4,85 | O 5,79 % |

4B — Stade 1 : (+) N-formyl-2-(3,4-dichlorobenzyl)alanine

Intermédiaire IV $R_1$ = $CH_3$ ; $R_4$ = H.

Le produit est préparé à partir de (+) 2-(3,4-dichlorobenzyl)alanine selon le mode opératoire de l'exemple 4A-stade 1. avec un rendement de 70,6 %. $^1$H-RMn (DMSO-$CDCl_3$) 1,45 (s, 3H) ; 3,30 (9, 2H) ; 7,05-7,65 (m, 4H dont 1 échang.) ; 8,10 (d, 1H) ; 10,35 (s large, 1H échang.).

Stade 2 : (+) 2-méthyl-2-méthylamino-3-(3,4-dichlorophényl)propanol (JO 1308)

Formule I $R_1 = R_2 = CH_3$, $R_3$ = H.

19,0 g du produit obtenu au stade précédent (69 mmol) sont réduits par le complexe BMS selon le mode opératoire décrit au stade 2 de l'exemple 4-A. Le produit brut est purifié par cristallisation dans le chlorure de méthylène.

Poids : 10,7 g — Rendement = 62,5 %
F = 113 °C — CCM : A ; 0,5

$[\alpha]_D^{20}$ = + 1,8° (C = 5 %, méthanol).
$^1$H-RMN (DMSO-$CDCl_3$) identique au produit racémique de l'exemple 4A.

Exemple 5

Stade 1 : (±) N-formyl 2-pentyl-2-(3,4 dichlorobenzyl)glycine

Intermédiaire IV $R_1 = (CH_2)_4CH_3$ ; $R_4$ = H.

Le produit est préparé selon l'exemple 4 — stade 1 à partir de 50,0 g (0,164 mol) de (±) 2-pentyl-2-(3,4 dichlorobenzyl)glycine.

Poids : 47,8 g — Rendement = 87,7 %
CCM : A ; 0,4.

Stade 2 : (±) 2-pentyl-2-méthylamino-3(3,4-dichlorophenyl)propanol (JO 1563)

Formule I — $R_1 = (CH_2)_4$—$CH_3$ ; $R_2 = CH_3$, $R_3$ = H.

Selon la méthode de l'exemple 4 — stade 2, on obtient le produit par traduction de 47,4 g (0,143 mol) du dérivé obtenu au stade 1 précédent.

Poids : 33,0 g — Rendement = 75,8 %
CCM : A ; 0.75.

$^1$H-RMN ($CDCl_3$) : 0,95 (t, 3H) ; 1,30 (s, 8H) ; 1,85 (s, 2H, échang.) ; 2,35 (s, 3H) ; 2,65 (s, 2H) ; 3,25 (5s, 2H) ; 6,95-7,50 (m, 3H).

Chlorhydrate — Rendement = 53,2 % — F = 151 °C (acétate éthyle).
Analyse $C_{15}H_{24}Cl_3NO$

| | | | | | |
|---|---|---|---|---|---|
| Calculée : | C 52,87 | H 7,10 | Cl 31,22 | N 4,11 | O 4,70 % |
| Trouvée : | C 52,81 | H 7,14 | Cl 31,06 | N 4,08 | O 4,61 % |

Exemple 6

La réduction des (±), (+) et (—) N-acétyl-2-(3,4-dichlorobenzyl)alanine par le complexe BMS permet d'obtenir les aminoalcools correspondants des exemples 6A, 6B et 6C en utilisant le mode opératoire décrit au stade 2 des exemples 4A et B.

6A — (±) 2-méthyl-2-éthylamino-3-(3,4-dichlorophényl)propanol (JO 1277).

Formule I : $R_1 = CH_3$ ; $R_2$ = H ; $R_3 = C_2H_5$.
Rendement = 71,7 % CCM : A ; 0,6
F = 111 °C (chlorure de méthylène).
$^1$H-RMN (DMSO-$CDCl_3$) 0,85-1,20 (m, 6H) ; 2,40-2,85 (m, 4H) ; 2,90-3,15 (m, 2H échang.) ; 3,25 (s, 2H) ; 6,90-7,40 (m, 3H).

Chlorhydrate — Rendement = 74,9 % F = 245 °C (éthanol).
Analyse $C_{12}H_{18}Cl_3NO$

| | | | | | |
|---|---|---|---|---|---|
| Calculée : | C 48,26 | H 6,08 | Cl 35,62 | N 4,69 | O 5,36 % |
| Trouvée : | C 48,32 | H 6,01 | Cl 35,63 | N 4,58 | O 5,45 % |

6B — (+) 2-méthyl-2-éthylamino-3-(3,4-dichlorophényl)propanol (JO 1295)

Formule I : $R_1$ = $CH_3$ ; $R_2$ = H ; $R_3$ = $C_2H_5$.
Rendement = 55,6 % CCM = A ; 0,6
F = 105 °C (éther).
$^1$H-RMN (DMSO-$CDCl_3$) identique au produit de l'exemple 6A.

Chlorhydrate — Rendement = 82,2 %  F = 268 °C (méthanol)
$[\alpha]_D^{20}$ = + 4,2° (C = 1 %, eau).
Analyse $C_{12}H_{18}Cl_3NO$

| | | | | | |
|---|---|---|---|---|---|
| Calculée : | C 48,26 | H 6,08 | Cl 35,62 | N 4,69 | O 5,36 % |
| Trouvée : | C 48,22 | H 6,02 | Cl 35,58 | N 4,63 | O 5,19 % |

6C — (—) 2-méthyl-2-éthylamino-3-(3,4-dichlorophényl)propanol (JO 1296).

Formule I : $R_1$ = $CH_3$ ; $R_2$ = H ; $R_3$ = $C_2H_5$.
Rendement = 67,9 %  CCM : A ; 06
F = 107 °C (éther).
$^1$H-RMN (DMSO-$CDCl_3$) identique au produit de l'exemple 6A.

Chlorhydrate — Rendement = 75,6 %  F = 269 °C (méthanol-éther)
$[\alpha]_D^{20}$ = — 5° (C = 1 %, eau).
Analyse $C_{12}H_{18}Cl_3NO$

| | | | | | |
|---|---|---|---|---|---|
| Calculée : | C 48,26 | H 6,08 | Cl 35,62 | N 4,69 | O 5,36 % |
| Trouvée : | C 48,16 | H 6,13 | Cl 35,75 | N 4,53 | O 5,31 % |

## Exemple 7

(±) 2-méthyl-2-allylamino-3-(3,4-dichlorophényl)propanol (JO 1453)

Formule I : $R_1$ = $CH_3$, $R_2$ = H ; $R_3$ = $CH_2$—CH = $CH_2$

Dans un réacteur protégé de l'humidité et sous agitation 30,0 g (128 mmol) de (±) 2-méthyl-2-amino-3-(3,4-dichlorophényl)propanol (JO 1275 — ex. 1A) et 14,0 g (116 mmol) de bromure d'allyle sont mis en suspension dans 300 ml d'acétonitrile. Le mélange est maintenu 1 heure à la température ambiante, puis chauffé 2 heures 30 à 50 °C.

Après refroidissement, l'insoluble est filtré et mis en suspension dans 2 l d'eau déminéralisée, alcanisé avec de la lessive de soude et le mélange est extrait 3 fois par 750 ml d'éther. Les phases éthérées réunies sont traitées de la façon habituelle. Après évaporation de l'éther, le résidu de 13,8 g est cristallisé dans 1 l d'hexane. Le produit est filtré, puis séché sous vide.

Poids : 11,2 g  Rendement = 31,9 g
F = 106 °C  CCM : B, 0,4.

$^1$H-RMN ($CDCl_3$) 1,00 (s, 3H) ; 1,90 (s large, 2H échang.) ; 2,65 (s, 2H) ; 3,10-3,35 (m, 4H) ; 5,15 (t, 2H) ; 5,60-6,25 (m, 1H) ; 6,90-7,45 (m, 3H).

Chlorhydrate — Rendement = 94,7 %  F = 230 °C (éthanol-éther).
Analyse $C_{13}H_{18}Cl_3NO$

| | | | | | |
|---|---|---|---|---|---|
| Calculée : | C 50,26 | H 5,84 | Cl 34,24 | N 4,51 | O 5,15 % |
| Trouvée : | C 50,22 | H 5,75 | Cl 34,33 | N 4,44 | O 5,00 % |

## Exemple 8

8A — (±) N-cyclopropanecarboxy-2-méthyl-2-amino-3-(3,4-dichlorophényl)propanol

Formule I : $R_1$ — $CH_3$ ; $R_2$ = H ;

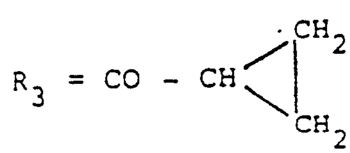

A une solution de 5,0 g (21,3 mmol) d'aminoalcool de l'exemple 1A (JO 1275) dans 100 ml de 1,2-dichloroéthane, on ajoute 0,57 g (14,2 mmol) d'hydroxyde de sodium en pastilles dissous dans 25 ml d'eau.

Le mélange est agité énergétiquement et refroidi à 5 °C, 1,48 g (14,2 mmol) du chlorure d'acide

cyclopropanecarboxylique sont alors ajoutés goutte à goutte en 30 minutes et à une température inférieure à 10 °C. L'agitation est maintenue durant 1 heure à 5-10 °C, puis la phase organique est séparée et extraite par 20 ml d'acide chlorhydrique normal, puis traitée de façon habituelle.

Après séparation du solvant, le produit est obtenu sous forme d'une huile jaune et visqueuse.

Poids : 4,3 g Rendement = 66,6 %
CCM : B ; 0,65-0,75.

$^1$H-RMN ($CDCl_3$) 0,55-1,65 (m, 7H) ; 3,05 (q, 2H) ; 3,55-3,85 (m, 3H) ; 5,10 (q, 1H échang.) ; 5,55 (s, large, 14) ; 6,90-7,45 (m, 3H).

8B — (±) 2-méthyl-2-cyclopropaneméthylamino-3-(3,4-dichlorophényl)propanol (JO 1454)

Formule : $R_1$ = $CH_3$ ; $R_2$ = H ;

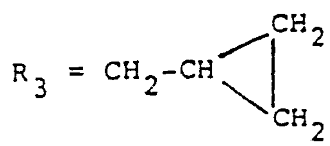

Dans un réacteur protégé de l'humidité 4,2 g (13,9 mmol) d'amidoalcool de l'exemple 8A sont dissous dans 60 ml de THF. Sous atmosphère d'azote on introduit de la manière habituelle 3,16 g (41,7 mmol) de BMS, puis on chauffe le mélange 4 heures 30 au reflux. Après refroidissement, on ajoute successivement 6,0 ml de méthanol puis 6,0 ml de solution d'hydroxyde de sodium N.

Après filtration de l'insoluble, le résidu obtenu par la concentration du filtrat est repris par 100 ml d'acide chlorhydrique 2N. La suspension acide est extraite deux fois par 50 ml d'éther, puis alcalinisée à pH12 avec de la lessive de soude et à nouveau extraite de l'éther. Les phases éthérées sont réunies et traitées de façon habituelle. Après évaporation, on obtient l'aminoalcool sous forme d'une huile visqueuse.

Poids : 2,9 g Rendement = 72,4 %
CCM : B, 0,25-0,4

$^1$H-RMN ($CDCl_3$) 0,05-1,25 (m, 7H) ; 2,05 (s large ; 2H échang.) ; 2,45 (d, 2H) ; 2,65 (s, 2H) ; 3,20 (s, 2H) ; 6,90-7,45 (m, 3H).

Chlorhydrate — Rendement = 86,3 % F = 237 °C (éthanol-éther).
Analyse $C_{14}H_{20}Cl_3NO$

| | | | | | |
|---|---|---|---|---|---|
| Calculée : | C 51,79 | H 6,21 | Cl 32,76 | N 4,31 | O 4,93 % |
| Trouvée : | C 51,87 | H 6,10 | Cl 32,69 | N 4,27 | O 4,83 % |

## Exemple 9

9A — (±) N-pivalyl-2-méthyl-2-amino-3-(3,4-dichlorophényl)propanol

Formule I : $R_1$ = $CH_3$ ; $R_2$ = H ; $R_3$ = $-CO-C(CH_3)_3$.

Dans une suspension de 5,0 g (21,3 mmol) d'aminoalcool de l'exemple 1A (JO 1275) dans 30,0 ml de toluène, on ajoute 3,7 g (36 mmol) de triéthylamine.

A 4 °C, sous agitation, une solution de 2,53 g (21 mmol) de chlorure de pivaloyle dans 30,0 ml de toluène est ajoutée en 1 heure. L'agitation est maintenue une heure après introduction, puis la solution est extraite deux fois par 25 ml d'acide chlorhydrique N. La phase toluénique est extraite à nouveau par 2 fois 25 ml de solution saturée en bicarbonate de sodium, puis traitée de la manière habituelle. Après élimination du toluène, le produit est obtenu sous forme d'une huile visqueuse jaune pâle.

Poids : 6,0 g Rendement = 89,7 %
CCM : B, 0,6-0,7

$^1$H-RMN ($CDCl_3$) 0,90-1,25 (m, 12H) ; 2,95 (q, 2H) ; 3,60 (d, 2H) ; 4,90 (t, 1H échang.) ; 5,40 (s large, 1H) ; 6,85-7,40 (m, 3H).

9B — (±) 2-méthyl-2-(2,2-diméthyl) propylamino-3-(3,4-dichlorophényl)propanol (JO 1455)

Formule I : $R_1$ = $CH_3$ ; $R_2$ = H ; $R_3$ = $-CH_2-C(CH_3)_3$

Selon le mode opératoire décrit à l'exemple 8B, 5,4 g (17 mmol) de l'amide précédemment obtenu sont réduits par 2,6 g (34 mmol) de complexe BMS.

Poids : 4,5 g — Rendement = 87,0 %
F = 91 °C (hexane) — CCM : B, 0,45-0,55 %

$^1$H-RMN ($CDCl_3$) 0,95 (s, 12H) ; 1,30-2,05 (m large, 2H échang.) ; 2,25 (s, 2H) ; 2,65 (s, 2H) ; 3,25 (s, 2H) ; 6,90-7,45 (m, 3H).

Chlorhydrate — Rendement = 71,5 % F = 225 °C (méthanol-éther).
Analyse $C_{15}H_{24}Cl_3NO$

| | | | | | |
|---|---|---|---|---|---|
| Calculée : | C 52,87 | H 7,10 | Cl 31,22 | N 4,11 | O 4,69 % |
| Trouvée : | C 53,02 | H 6,98 | Cl 31,15 | N 4,09 | O 4,57 % |

Exemple 10

10A — (±) N-phénylacétyl-2-méthyl-2-amino-3-(3,4-dichlorophényl)propanol

Formule I : $R_1$ = $H_3$ ; $R_2$ = H ; $R_3$ = $CO—CH_2—C_8—H_5$
Le produit est préparé selon le mode opératoire décrit à l'exemple 8A par réaction entre 2,2 g (14,2 mmol) de chlorure d'acide phénylacétique et de 5,0 g (21,3 mmol) d'aminoalcool obtenu à l'exemple 1A (JO 1275).

Poids : 4,9 g — Rendement = 65,3 %
CCM : C, 0,80

$^1$H-RMN ($CDCl_3$) 1,05 (s, 3H) ; 2,90 (q, 2H) ; 3,55 (d, 2H) ; 4,70 (s, 2H) ; 4,90 (s large, 1H échang.) ; 5,45 (s large, 1H) ; 6,70-7,50 (m, 8H).

10B — (±) 2-méthyl-2-(2-phényl) éthylamino-3-(3,4-dichlorophényl)propanol (JO 1456)

Formule I : $R_1$ = $CH_3$ ; $R_2$ = H ; $R_3$ = $—CH_2—CH_2—C_6H_5$
Obtenu selon le mode opératoire de l'exemple 8B par réduction de 4,8 g (13,6 mmol) de l'alcool amide précédemment décrit avec 3,1 g (40,8 mmol) de complexe BMS.

Poids : 2,9 g — Rendement = 63,0 %
F = 113 °C (hexane) — CCM : B, 0,6-0,7.

$^1$H-RMN ($CDCl_3$) 0,90 (s, 3H) ; 1,3-2,2 (m large, 2H échang.) ; 3,55 (s, 2H) ; 3,75 (s, 4H) ; 3,15 (s, 2H) ; 6,75-7,35 (m, 8H).

Chlorhydrate — Rendement = 84,0 % F= 235°C (acétate d'éthyle).
Analyse $C_{18}H_{22}Cl_3NO$

| | | | | | |
|---|---|---|---|---|---|
| Calculée : | C 57,29 | H 5,92 | Cl 28,38 | N 3,74 | O 4,27 % |
| Trouvée : | C 57,65 | H 5,84 | Cl 28,30 | N 3,68 | O 4,22 % |

Exemple 11

(±) 2-méthyl-2-(N-méthyl, N-éthyl) amino-3-(3,4-dichlorophényl)propanol (JO 1291)

Formule I : $R_1$ = $CH_3$ ; $R_2$ = $CH_3$ ; $R_3$ = $C_2H_5$
Sur bain-marie à 50 °C environ, 20,0 g (76,3 mmol) d'aminoalcool de l'exemple 6A (JO 1277) sont mélangés intimement avec 17,6 ml de solution de formaldéhyde à 15 °C et 12,9 ml d'acide formique pur (d = 1,22) sont ajoutés peu à peu, le mélange devient alors moins visqueux et on obtient une solution qui est chauffée 1 heure 30 à 100 °C.
Après refroidissement, 100 ml d'eau glacée sont ajoutés, la solution est acidifiée jusqu'à pH 1 avec de l'acide chlorhydrique concentré, puis extraite 3 fois par 50 ml d'éther.
La phase acide est alcalinisée avec une solution concentrée d'hydroxyde de sodium, puis extraite 3 fois par 75 ml d'éther. Les phases éthérées sont traitées de façon habituelle et le résidu huileux obtenu après évaporation de l'éther est cristallisé dans 150 ml d'hexane. Le produit blanc cristallisé est filtré et séché.

Poids : 19,45 g — Rendement = 92,3 %
F = 55 °C.

$^1$H-RMN ($CDCl_3$) 0,95 (s, 3H) ; 1,10 (t, 3H) ; 2,30 (s, 3H) ; 2,55 (d, 2H) ; 2,65 (s, 2H) ; 3,00 (s large, 1H échang.) ; 3,25 (d, 2H) ; 6,90-7,45 (m, 3H).

Chlorhydrate — Rendement = 64,4 % F = 192 °C (éthanol).
Analyse $C_{13}H_{20}Cl_3NO$
Calculée : C 49,94 H 6,45 Cl 34,02 N 4,48 O 5,12 %
Trouvée : C 49,83 H 6,35 Cl 33,93 N 4,38 O 5,21 %

Exemple 12

(±) 2-méthyl-2-[N-méthyl, N-(2-phényl]éthyllamino-3-(3,4-dichlorophényl)propanol (JO 1457)

Formule I : $R_1 = CH_3$ ; $R_2 = CH_3$ ; $R_3 = CH_2—CH_2—C_6H_5$
Le produit est préparé selon le mode opératoire décrit à l'exemple précédent, à partir de 8,9 g (26,3 mmol) de l'aminoalcool préparé à l'exemple 10B (JO 1456). Le produit se présente sous forme d'une huile visqueuse.

Poids : 8,8 g Rendement = 95,0 %
CCM = C, 0,55.

$^1$H-RMN ($CDCl_3$) 0,85 (s, 3H) ; 2,20 (s large, 1H échang.) ; 2,25 (s, 3H) ; 2,40-2,75 (m, 6H) ; 3,10 (d, 2H) ; 6,85-7,35 (m, 8H).

Chlorhydrate — Rendement = 65,3 % F = 212 °C (éthanol-éther).
Analyse $C_{19}H_{24}Cl_3NO$
Calculée : C 58,70 H 6,22 Cl 27,36 N 3,60 O 4,11 %
Trouvée : C 58,62 H 6,17 Cl 27,31 N 3,57 O 4,03 %

Exemple 13

(±) 2-méthyl-2-(N-méthyl, N-allyl)amino-3-(3,4-dichlorophenyl)propanol (JO 1581)

Formule I — $R_1 = (CH_3)$ ; $R_2 = R_3 = CH_2—CH = CH_2$
Le produit est préparé selon le mode opératoire de l'exemple 11 précédent à partir de 3,8 g (13,8 mmol) de l'amino alcool obtenu à l'exemple 7. Le produit est obtenu sous forme d'une huile jaune pâle, visqueuse.

Poids : 3,8 g Rendement = 95,5 %
CCM : B ; 0,60.

$^1$H-RMN ($CDCl_3$) : 0,95 (s, 3H) ; 2,25 (s, 3H) ; 2,70 (s, 2H) ; 2,90 (s, 1H échang.) ; 3,15 (d, 2H) ; 3,25 (d, 2H) ; 5,25 (d, 2H) ; 5,55-5,95 (m, 1H) ; (6,95-7,45 (m, 3H).

Chlorhydrate — Rendement = 86,5 % — F = 190 °C (acétate d'éthyle).
Analyse $C_{14}H_{20}Cl_3NO$
Calculée : C 51,79 H 6,21 Cl 32,76 N 4,31 O 4,93 %
Trouvée : C 51,73 H 6,21 Cl 32,68 N 4,26 O 5,05 %

Les produits des exemples 14A, 14B et 14C sont respectivement obtenus à partir des aminoalcools des exemples 1A (JO 1275, 1B (JO 1307) et 1C par réaction avec le formaldéhyde et l'acide formique. Il en est de même pour les produits des exemples 15 et 16 qui sont respectivement préparés à partir des amino alcools des exemples 2 et 3.

Mode opératoire

A 0,1 mole d'aminoalcool on ajoute 24,6 ml de solution de formaldéhyde à 37 % (p/v) soit 9,0 g (0,3 mol). Les réactifs sont énergiquement mélangés en tiédissant au besoin pour obtenir une gomme homogène à laquelle on ajoute peu à peu et en refroidissant si nécessaire, 18,9 ml (0,5 mol) d'acide formique à 99-100 % (d = 1,22). Le mélange visqueux est porté 1 heure 30 sur bain-marie bouillant, puis refroidi à 20 °C et additionné avec 125 ml d'eau.

La solution est acidifiée jusqu'à pH 1 par de l'acide chlorhydrique concentré et extraite trois fois par 50 ml d'éther. Les phases éthérées sont écartées et la phase acide alcalinisée jusqu'à pH 12 par une solution concentrée d'hydroxyde de sodium, puis extraite à nouveau par 3 fois 75 ml d'éther.

Les phases éthérées réunies sont traitées de façon habituelle.

Exemple 14

14A — (±) 2-méthyl-2-diméthylamino-3-(3,4-dichlorophanyl)propanol (JO 1017)

Formule I : $R_1 = R_2 = R_3 = CH_3$ cristaux blancs.
Rendement = 79,2 % F = 88 °C (hexane)
CCM : A, 0,4-0,5.

$^1$H-RMN ($CDCl_3$) 0,90 (s, 3H) ; 2,30 (s, 6H) ; 2,65 (s, 2H) ; 3,10 (s large, 1H échang.) ; 3,20 (s, 2H) ; 6,95-7,45 (m, 3H).

Chlorhydrate — Rendement = 84,0 % F = 177 °C (éthanol).
Analyse $C_{12}H_{18}Cl_3NO$

| | C | H | Cl | N | O |
|---|---|---|---|---|---|
| Calculée : | 48,26 | 6,07 | 35,62 | 4,69 | 5,36 % |
| Trouvée : | 48,35 | 6,04 | 35,47 | 4,70 | 5,43 % |

14B — (—) 2-méthyl-2-diméthylamino-3-(3,4-dichlorophényl)propanol (JO 1239)

Formule I : $R_1 = R_2 = R_3 = CH_3$.
Huile — Rendement = 53,1 % CCM : A, 0,4-0,5
$[\alpha]_D^{20}$ = — 6,5° (C = 1 % éthanol).

$^1$H-RMN ($CDCl_3$) identique au produit de l'exemple 14A.

Chlorhydrate — Rendement = 79,2 % F = 192 °C (éthanol).
Analyse $C_{12}H_{18}Cl_3NO$

| | C | H | Cl | N | O |
|---|---|---|---|---|---|
| Calculée : | 48,26 | 6,07 | 35,62 | 4,69 | 5,36 % |
| Trouvée : | 48,21 | 6,09 | 35,55 | 4,63 | 5,39 % |

14C — (+) 2-méthyl-2-diméthylamino-3-(3,4-dichlorophényl)propanol (JO 1240)

Formule I : $R_1 = R_2 = R_3 = CH_3$.
Huile — Rendement = 66,5 % CCM : A, 0,4-0,5
$[\alpha]_D^{20}$ = + 6,2° (C = 2 % éthanol).

$^1$H-RMN ($CDCl_3$) identique au produit des exemples 14A et B.

Chlorhydrate — Rendement = 79,7 % F = 193 °C (éthanol).
Analyse $C_{12}H_{18}Cl_3NO$

| | C | H | Cl | N | O |
|---|---|---|---|---|---|
| Calculée : | 48,26 | 6,07 | 35,62 | 4,69 | 5,36 % |
| Trouvée : | 48,16 | 6,12 | 35,61 | 4,57 | 5,45 % |

## Exemple 15

(±) 2-isopropyl-2-dimethylamino-3(3,4-dichlorophenyl)propanol (JO 1580)

Formule I — $R_1 = CH(CH_3)_2$ ; $R_2 = R_3 = CH_3$
Huile — Rendement = 96,6 % — CCM : A, 0,65

$^1$H-RMN ($CDCl_3$) : 0,80-1,20 (m, 6H) ; 1,65-2,20 (m, 1H) ; 2,40 (s, 6H) ; 2,75 (s, 2H) ; 3,20 (s, 1H échang.) ; 3,45 (s, 2H) ; 7,00-7,45 (m, 3H).

Chlorhydrate — Rendement = 92,9 % — F = 178 °C (acétone)
Analyse $C_{14}H_{22}Cl_3NO$

| | C | H | Cl | N | O |
|---|---|---|---|---|---|
| Calculée : | 51,47 | 6,79 | 32,56 | 4,29 | 4,89 % |
| Trouvée : | 51,56 | 6,59 | 32,53 | 4,26 | 4,98 % |

## Exemple 16

(±) 2-pentyl-2-dimethylamino-3(3,4-dichlorophenyl)propanol (JO 1564)

Formule I — $R_1 = (CH_2)_4CH_3$ ; $R_2 = R_3 = CH_3$
Huile — Rendement = 92,5 % — CCM : A, 0,65
$^1$H-RMN ($CDCl_3$) : 0,80 (t, 3H) ; 1,30 (s, 8H) ; 2,35 (s, 6H) ; 2,65 (s, 2H) ; 3,25 (s, 3H dont 1H échang.) ; 6,85-7,35 (m, 3H)

Chlorhydrate — Rendement = 92,7 % — F = 160 °C (acétate d'éthyle).

Analyse $C_{16}H_{26}Cl_3NO$
Calculée : C 54,17 H 7,38 Cl 29,98 N 3,95 O 4,51 %
Trouvée : C 54,15 H 7,26 Cl 29,82 N 3,88 O 4,60 %

## Exemple 17

(±) 2-méthyl-2-(1-pipéridyl-3-(3,4-dichlorophényl)propanol (JO 1467)

Formule I : $R_1 = CH_3$ ; $NR_2 \cdot R_3$ = pipéridyle.

Dans un réacteur protégé de l'humidité, 5 g (21,3 mmol) de l'aminoalcool obtenu à l'exemple IA, 5,14 g (22,3 mmol) de 1,5-dibromopentane sont dissous dans 40 ml de toluène.

La solution est portée au reflux sous agitation durant une heure puis refroidie à la température ambiante ; 4,58 g (43,2 mmol) de carbonate de sodium sont alors ajoutés et la suspension obtenue est portée à nouveau au reflux durant 20 heures.

Après refroidissement, 100 ml d'acide chlorhydrique 2N sont ajoutés lentement, l'insoluble est filtré, lavé au toluène.

Le filtrat est éliminé, l'insoluble est repris par 500 ml d'eau. La suspension obtenue est alcalinisée jusqu'à pH 12 par addition de solution d'hydroxyde de sodium 10N. Le mélange est extrait à 3 reprises par 250 ml d'éther. Les phases éthérées réunies sont traitées de façon habituelle. Après évaporation, le produit est obtenu sous forme de solide blanc et amorphe.

Poids : 2,2 g — Rendement = 34,2 %
F = 94 °C — CCM : A ; 0,6.

$^1$H-RMN ($CDCl_3$) : 0,95 (s, 3H) ; 1,55 (s large, 6H) ; 2,50-2,75 (m, 6H) ; 2,90 (s, H échang.) ; 3,30 (q, 2H) ; 6,90-7,45 (m, 3H).

Analyse $C_{15}H_{21}Cl_3NO$
Calculée : C 59,61 H 7,00 Cl 23,46 N 4,63 O 5,29 %
Trouvée : C 59,42 H 6,99 Cl 23,58 N 4,74 O 5,30 %

## Exemple 18

(±) 2-méthyl-2-(4-morpholinyl)-3-(3,4-dichlorophenyl)propanol (JO 1565)

Formule I — $R_1 = CH_3$ ; $NR_2—R_3$ = morpholine

Dans un réacteur protégé de l'humidité, 23,4 g (0,1 mol) de l'amino alcool obtenu à l'exemple 1A sont dissous dans 300 ml de diméthylformamide.

Sous agitation 15,7 g (0,110 mol) de bis-2-chloroéthyléther puis 18,5 g (0,220 mol) de bicarbonate de sodium sont ajoutés.

La suspension est maintenue sous agitation à 100 °C durant 48 heures. L'insoluble est filtré, le diméthylformamide éliminé par distillation sous vide.

Le résidu est repris par l'éther, la solution extraite à 3 reprises par successivement 150-100 et 100 ml de solution HCl N.

Les phases chlorhydriques réunies sont alcalinisées par addition de lessive de soude jusqu'à pH 12. Le mélange est ensuite extrait à l'éther. Les phase éthérées réunies sont lavées par une solution saturée en chlorure de sodium puis desséchées sur $Na_2SO_4$. L'éther est éliminé par distillation.

Poids du résidu = 33,0 g.

Ce résidu est purifié par chromatographie sur colonne de silice. L'élution par un mélange chlorure de méthylène-méthanol 95-5 (v/v) permet d'obtenir le produit.

Poids = 14,5 g — Rendement = 47,7 %
CCM : C, 0,75

Chlorhydrate — Rendement = 76,9 % — F = 232 °C (méthanol-éther).
Analyse $C_{14}H_{20}Cl_3NO_2$
Calculée : C 49,36 H 5,92 Cl 31,22 N 4,11 O 9,39 %
Trouvée : C 49,38 H 5,88 Cl 31,17 N 4,03 O 9,49 %

## Exemple 19

(±) 2-méthyl-2-(1-(4-méthyl)pipérazinyl)-3-(3,4-dichlorophenyl)propanol (JO 1566)

Le produit est obtenu selon le mode opératoire de l'exemple 18 précédent, à partir de 46,8 g (0,200 mol) de l'amino alcool de l'exemple 1A, de 42,2 g (0,220 mol) de chlorhydrate de N-méthyl bis (2-chloroéthyl)amine et de 54 g (0,640 mol) de bicarbonate de sodium.

Poids = 6,2 g Rendement = 9,8 %
F = 118 °C (acétate d'éthyle) CCM : D, 0,45

Analyse $C_{15}H_{22}Cl_2N_2O$
Calculée : C 56,71 H 6,99 Cl 22,35 N 8,83 O 5,04 %
Trouvée : C 56,73 H 6,82 Cl 22,28 N 8,74 O 5,20 %
$^1$H-RMN ($CDCl_3$) : 1,05 (s, 3H) ; 2,40 (s, 3H) ; 2,45-3,05 (m, 10H) ; 3,05-3,65 (m, 3H dont 1H échang.) ; 6,95-7,50 (m, 3H).

Poids : 2,2 g Rendement = 34,2 %
F = 94 °C CCM : A ; 0,6.

$^1$H-RMN ($CDCl_3$) : 0,95 (s, 3H) ; 1,55 (s large, 6H) ; 2,50-2,75 (m, 6H) ; 2,90 (s, H échang.) ; 3,30 (q, 2H) ; 6,90-7,45 (m, 3H).

Analyse $C_{15}H_{21}Cl_2NO$
Calculée : C 59,61 H 7,00 Cl 23,46 N 4,63 O 5,29 %
Trouvée : C 59,42 H 6,99 Cl 23,58 N 4,74 O 5,30 %

L'étude pharmacologique des produits de l'invention montre d'intéressantes propriétés, en particulier une activité sur le système nerveux central (S.N.C.) accompagnée de propriétés analgésiques. Par ailleurs, leur toxicité a été déterminée. L'ensemble de ces études est rapporté dans la partie qui suit et les résultats obtenus démontrent l'activité thérapeutique de ces produits, ainsi que leur utilité comme médicaments.

La toxicité aiguë a été étudiée par voie orale sur la souris mâle. Pour cela, les produits ont été administrés en solution aqueuse à raison de 2 ml/100 g. Les animaux ont été ensuite observés durant les trois heures qui suivent l'administration puis quotidiennement durant quatorze jours, moment auquel ils ont été sacrifiés et autopsiés.

Les $DL_{50}$ (doses léthales provoquant la mort de 50 % des animaux) ont été calculées selon la méthode de Reed J. L. et Muench H. (Am. J. Hyg. 1939, 27, 493). Leur valeur est généralement comprise entre 750 et 1 200 mg-$kg^{-1}$ et parfois supérieure à ce dernier chiffre.

Activité sur le S.N.C.

Cette activité a été recherchée sur les souris par le test de la suspension par la queue selon la technique décrite par Thierry et coll. (Behavioral and Neural Biology, 1984, 41, 180-189), test qui a été proposé comme modèle chez l'animal, pour sélectionner les produits actifs sur le S.N.C.

Pratiquement, les essais ont été réalisés sur des lots de 10 souris mâles pour chaque produit et à chaque dose, les substances à étudier étant administrées en solution aqueuse, par voie orale, une heure avant le test, un lot témoin d'animaux ne recevant que de l'eau distillée.

Les résultats des essais sont exprimés en pourcentages d'activité par rapport au lot d'animaux témoins.

Activité analgésique

Elle a été recherchée et déterminée sur la souris par le test à l'acide acétique réalisé selon une technique dérivée de celle de Troster R. (Fed. Proc. 1959, 18, 412).

Vingt heures avant le test, les animaux sont mis à jeun de nourriture et de boisson. Ils reçoivent alors par voie orale et en solution aqueuse les produits à étudier à raison de 2 ml de solution par 100 g de poids corporel, puis 30 minutes après une injection intrapéritonéale de 0,25 ml d'une solution d'acide acétique à 0,25 % (p/v) maintenue à 37 °C.

Trois minutes après cette injection, le nombre de crampes abdominales présentées par les animaux commence à être compté et ce durant 10 minutes.

Les animaux sont considérés comme analgésiés, lorsqu'ils présentent un nombre de crampes inférieur à la moitié de la moyenne des crampes présentées par les animaux du lot témoin.

Les produits ont été administrés à la dose de 30 mg-$kg^{-1}$ et les résultats sont présentés en pourcentage d'animaux analgésiés. Pour certains produits, plusieurs doses ont été administrées et le calcul de leur $DE_{50}$ (dose provoquant l'analgésie de 50 % des animaux) a été déterminé en log-probit.

Les résultats de ces deux tests sont résumés dans les tableaux I et II qui suivent.

(Voir tableaux 1 et 2 pages suivantes)

Résultats des essais pharmacologiques

Tableau I

| Exemple N° | Code laboratoire | S.N.C. : Test de suspension | |
|---|---|---|---|
| | | Dose : 32 mg/kg | Dose : 128 mg/kg |
| 1-A | JO 1275 | - 78 % | - 86 % |
| 2 | JO 1579 | | - 67 % |
| 3 | JO 1562 | - 73 % | |
| 4-A | JO 1276 | - 58 % | - 83 % |
| 5 | JO 1563 | - 49 % | - 71 % |
| 11 | JO 1291 | - 58 % | |
| 12 | JO 1457 | - 60 % | |
| 13 | JO 1581 | | - 61 % |
| 14-A | JO 1017 | - 65 % | - 85 % |
| 14-B | JO 1239 | - 74 % | - 64 % |
| 14-C | JO 1240 | - 77 % | - 85 % |
| 16 | JO 1564 | | - 74 % |
| Clomipramine | | - 49 % | - 54 % |

Tableau II

| Exemple No. | Code laboratoire | Analgésie : Test à l'acide acétique | |
|---|---|---|---|
| | | Dose : 30 mg/kg | $DE_{50}$ mg/kg |
| 1-A | JO 1275 | -43 % | |
| 1-B | JO 1307 | -50 % | |
| 4-A | JO 1276 | -86 % | 8,5 |
| 4-B | JO 1308 | -88 % | |
| 6-A | JO 1277 | -43 % | |
| | | | |
| 3-C | JO 1296 | -38 % | |
| 11 | JO 1291 | -38 % | |
| 14-A | JO 1017 | -86 % | 14,0 |
| 14-B | JO 1239 | -86 % | 11,0 |
| 14-C | JO 1240 | -86 % | 13,0 |
| Noramydopyrine | | - | 20 à 23 |

Ainsi que le montrent ces résultats les produits de l'invention présentent sur le système nerveux central une activité aux doses de 32 et 128 $mg\text{-}kg^{-1}$ supérieure à celle de la clomipramine.

D'autre part les propriétés analgésiques des produits de l'invention sont clairement démontrées à la dose de 30 $mg\text{-}kg^{-1}$ par voie per os. Le calcul des $DE_{50}$ montre une supériorité d'activité par rapport à la Noramidopyrine, analgésique connu, pris comme substance de référence.

Les produits de l'invention montrent donc à la fois des propriétés psychotropes et analgésiques qui sont d'utilité clinique multiple sous la forme de médicaments destinés à la thérapeutique humaine ou vétérinaire.

Ces préparations ston, entre autres, particulièrement destinées aux traitements des troubles psychopathologiques et neuropathologiques, ainsi qu'aux syndromes douloureux d'étiologies diverses.

Ils sont applicables aux psychoses et névroses ayant pour exemples des symptômes tels que des modifications de l'humeur, de la mémoire, du tonus psychomoteur et de certaines fonctions organiques.

En outre, l'association des propriétés psychotropes et analgésiques des produits rend ces préparations adaptées aux traitements des insomnies, des céphalées, de certaines douleurs migratrices ou angineuses que de certains états nauséeux.

Les produits de l'invention, tels quels ou sous forme de leurs sels pharmaceutiquement acceptables sont administrés sous forme de compositions par des voies appropriées à la nature et à la gravité de l'affection à traiter et ce sous des formes compatibles aux voies d'administration envisagées.

La toxicité relativement faible des produits autorise une posologie journalière, chez l'homme, voisine de un gramme de produit. Plus habituellement cette posologie est comprise entre 0,010 et 0,500 gramme du produit par jour, cette quantité pouvant être répartie en plusieurs prises.

Les compositions pharmaceutiques selon l'invention comprennent de 1 à 40 % en poids de principe actif constitué par un ou plusieurs composés de formule (I) ou de leurs sels et 99 à 60 % en poids d'un véhicule pharmaceutique compatible avec la forme physique de la composition envisagée.

Les compositions sont préparées par des méthodes connues en soi et leur forme est compatible avec la voie d'administration. A titre d'exemples on cite les comprimés, dragées, capsules, poudres, les solutions injectables ou buvables, les suspensions, les gels et les suppositoires. Leur fabrication est illustrée de façon non limitative par la description des méthodes de préparation des comprimés et des solutions isotoniques injectables avec les principes actifs de l'invention.

Comprimés

— Formule

| | |
|---|---|
| Substance active selon l'exemple 10A | 5 à 75 mg |
| Polyvinylpyrrolidone | 2 mg |
| Carboxyméthylamidon | 8 mg |
| Stéarate de magnésium | 3 mg |
| Lactose | 60 à 76 mg |
| Cellulose monocristalline | 122 à 76 mg |

pour un comprimé de 200 mg.

Fabrication

Dissoudre la polyvinylpyrrolidone à une concentration comprise entre 0,1 et 1,0 % en poids dans l'eau, ou un alcool de bas poids moléculaire comme l'éthanol ou encore un mélange hydroalcoolique.

Mélanger intimement la substance active, la lactose, la moitié de la quantité de cellulose cristalline et du carboxyméthylamidon mis en œuvre puis humidifier ce mélange avec la solution de polyvinylpyrrolidone obtenue précédemment.

Granuler la pâte obtenue sur granulateur oscillant, puis sécher les granulés en étuve ou sur un lit d'air fluidisé.

Calibrer sur tamis les granulés séchés et ajouter le stéarate de magnésium, le reste de cellulose microcristalline et de carboxyméthylamidon. Mélanger intimement puis comprimer à raison de 200,0 mg par comprimé.

Soluté isotonique injectable

— Formule

| | |
|---|---|
| Substance active selon l'exemple IA (sous forme de chlorhydrate) | 10 mg |
| Chlorure de sodium | 9 mg |
| Eau distillée (quantité suffisante pour) | 1,0 ml |

Le soluté isotonique est réparti en ampoules de volume approprié qui sont, après scellement, stérilisées par des moyens thermiques connus en soi, ou bien le soluté est stérilisé par filtration, réparti en ampoules qui sont ensuite scellées, l'ensemble des opérations étant effectué sous atmosphère stérile.

Dans ce dernier cas, on préfère ajouter à la formule décrite, 1 % d'alcool benzylique à titre d'agent bactériostatique, soit 10 mg de cet alcool par ml de soluté.

**Revendications** (pour les Etats contractants : BE, CH, DE, FR, GB, IT, LI, LU, NL, SE)

1. Aminoalcools de formule

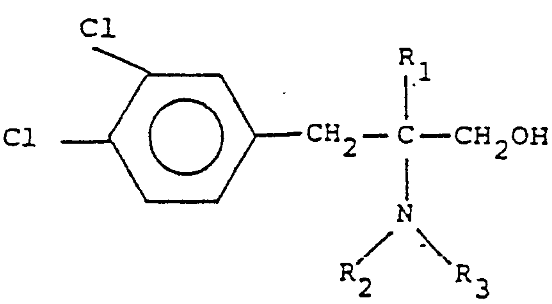

(I)

dans laquelle :

$R_1$ est alkyle inférieur,

$R_2$ est H ou alkyle inférieur,

$R_3$ est H, alkyle inférieur, alkényle inférieur, phénylalkyle inférieur ou cycloalkylalkyle inférieur ayant de 3 à 6 atomes de carbone dans le cycle, ou, quand $R_2$ est H, alcanoyle inférieur, phénylalcanoyle inférieur ou cycloalkylcarbonyle ayant de 3 à 6 atomes de carbone dans le cycle, ou $R_2$ et $R_3$ forment, ensemble avec l'atome d'azote auquel ils sont reliés, un hétérocycle saturé de cinq à sept chaînons pouvant comprendre, comme second hétéroatome non relié directement à l'atome d'azote, un oxygène, un soufre ou un azote, ce dernier hétéroatome d'azote pouvant porter un substituant alkyle en $C_1$ à $C_4$, et leurs sels d'addition avec des acides.

2. Aminoalcools suivant la revendication 1, caractérisés en ce que $R_1$ est méthyle.

3. Aminoalcools suivant la revendication 1 ou 2, caractérisés en ce que $R_2$ est hydrogène ou méthyle.

4. Aminoalcools suivant la revendication 1, 2 ou 3, caractérisés en ce que $R_3$ est hydrogène ou méthyle.

5. Le 2-méthyl-2-amino-3-(3,4-dichlorophényl)propanol et ses sels,

6. Le 2-méthyl-2-méthylamino-3-(3,4-dichlorophényl)propanol et ses sels,

7. Le 2-méthyl-2-éthylamino-3-(3,4-dichlorophényl)propanol et ses sels,

8. Le 2-méthyl-2-allylamino-3-(3,4-dichlorophényl)propanol et ses sels,

9. Le 2-méthyl-2-cyclopropaneméthylamino-3-(3,4-dichlorophényl)propanol et ses sels,

10. Le 2-méthyl-2-(2,2-diméthyl-propylamino-3-(3,4-dichlorophényl)propanol et ses sels,

11. Le 2-méthyl-2-(2-phényl)éthylamino-3-(3,4-dichlorophényl)propanol et ses sels,

12. Le 2-méthyl-2-(N-méthyl, N-éthyl)amino-3-(3,4-dichlorophényl)propanol et ses sels,

13. Le 2-méthyl-2-[N-méthyl, N-(2-phényl)éthyl]amino-3-(3,4-dichlorophényl)propanol et ses sels,

14. Le 2-méthyl-2-(N-méthyl, N-allyl) amino-3-(3,4-dichlorophényl) propanol et ses sels,

15. Procédé de préparation des composés suivant la revendication 1, caractérisé en ce qu'il consiste :

à réduire un acide de formule

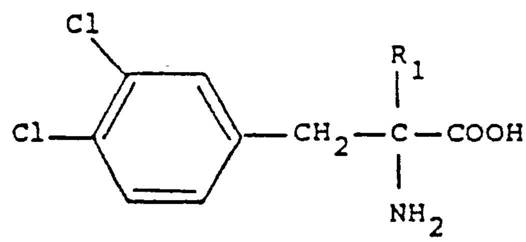

(II)

par un hydrure de bore ou d'aluminium pour obtenir les composés (Ia) dans lesquels $R_2$ et $R_3$ sont l'hydrogène, et

pour obtenir les composés (Ib), dans lesquels $NR_2R_3$ forment un hétérocyle, à effectuer une cyclisation par réaction d'un composé (Ia) sur un réactif de formule

$$X-(CH_2)_m-R_5-(CH_2)_n-X'$$

dans laquelle X et X', semblables ou différents, sont halogènes, $R_5$ est une liaison simple de valence et m et n sont des nombres entiers allant de 1 à 3, avec m + n supérieur ou égal à 4 et inférieur ou égal à 6, ou $R_5$ est un oxygène, un soufre ou $NR_6$, $R_6$ étant H ou alkyle en $C_1$ à $C_4$ et m et n sont des nombres entiers allant de 1 à 3 avec m + n supérieur ou égal à 3 et inférieur ou égal à 5, et

pour préparer les composés (Ic) dans lesquels $R_3$ est alkanoyle, phénylalkanoyle ou cycloalkylcarbonyle, à acyler un composé (Ia) par un agent d'acylation de formule $R_4$-COX dans laquelle X est un halogène et $R_4$ est l'homologue immédiatement inférieur de $R_3$, et

pour préparer les composés Id, dans lesquels $R_2$ est H et $R_3$ est alkyle, alkényle, phénylalkyle, ou cycloalkylalkyle, à alkyler un composé (Ia) par un agent d'alkylation $R_3$-X, X étant un halogène, ou à réduire un composé (Ic) par un hydrure de carbone ou d'aluminium, ou à réduire un composé de formule

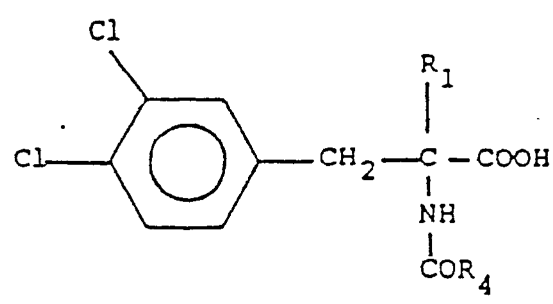

(IV)

par un hydrure de bore ou d'aluminium, et

pour préparer les composés (Ie), dans lesquels $R_2$ est alkyle et $R_3$ est identique à $R_2$, à faire réagir un composé (Ia) sur un aldéhyde de formule $R_4CHO$ en présence d'acide formique, et

pour préparer les composés (Ie) dans lesquels $R_2$ est alkyle et $R_3$ est alkyle, alkényle, phénylalkyle ou cycloalkylalkyle, à faire réagir un composé (Id) sur un aldéhyde de formule $R_4CHO$ en présence d'acide formique et, pour préparer les sels d'addition avec des acides, à faire réagir les composés (I) sur un acide.

16. Médicament analgésique et ayant une activité sur le système nerveux central, caractérisé en ce qu'il comprend, à titre de principe actif, un aminoalcool suivant l'une des revendications 1 à 14.

**Revendications** (pour les Etats contractants : AT, ES)

1. Procédé de préparation d'aminoalcools de formule

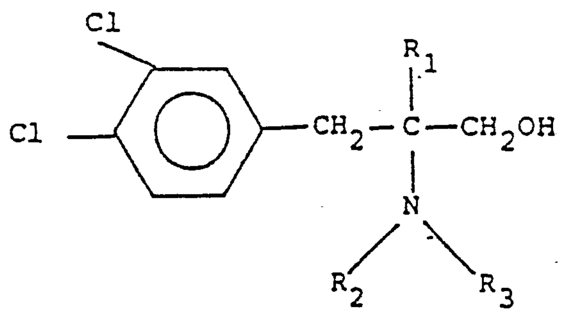

(I)

dans laquelle :

$R_1$ est alkyle inférieur,

$R_2$ est H ou alkyle inférieur,

$R_3$ est H, alkyle inférieur, alkényle inférieur, phénylalkyle inférieur ou cycloalkylalkyle inférieur ayant de 3 à 6 atomes de carbone dans le cycle, ou, quand $R_2$ est H, alcanoyle inférieur, phénylalcanoyle inférieur ou cycloalkylcarbonyle ayant de 3 à 6 atomes de carbone dans le cycle, ou $R_2$ et $R_3$ forment, ensemble avec l'atome d'azote auquel ils sont reliés, un hétérocycle saturé de cinq à sept chaînons pouvant comprendre, comme second hétéroatome non relié directement à l'atome d'azote, un oxygène, un soufre ou un azote, ce dernier hétéroatome d'azote pouvant porter un substituant alkyle en $C_1$ à $C_4$, et leurs sels d'addition avec des acides, caractérisé en ce qu'il consiste :

à réduire un acide de formule

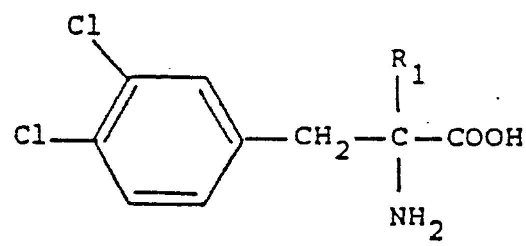

(II)

par un hydrure de bore ou d'aluminium pour obtenir les composés (Ia) dans lesquels $R_2$ et $R_3$ sont l'hydrogène, et

pour obtenir les composés (Ib), dans lesquels $NR_2R_3$ forment un hétérocycle, à effectuer une cyclisation par réaction d'un composé (Ia) sur un réactif de formule

$$X—(CH_2)_m—R_5—(CH_2)_n—X'$$

dans laquelle X et X', semblables ou différents, sont halogènes, $R_5$ est une liaison simple de valence et m et n sont des nombres entiers allant de 1 à 3, avec m + n supérieur ou égal à 4 et inférieur ou égal à 6, ou $R_5$ est un oxygène, un soufre ou $NR_6$, $R_6$ étant H ou alkyle en $C_1$ à $C_4$ et m et n sont des nombres entiers allant de 1 à 3 avec m + n supérieur ou égal à 3 et inférieur ou égal à 5, et

pour préparer les composés (Ic) dans lesquels $R_3$ est alkanoyle, phénylalkanoyle ou cycloalkylcarbonyle, à acyler un composé (Ia) par un agent d'acylation de formule $R_4$-COX dans laquelle X est un halogène et $R_4$ est l'homologue immédiatement inférieur de $R_3$, et

pour préparer les composés Id, dans lesquels $R_2$ est H et $R_3$ est alkyle, alkényle, phénylalkyle, ou cycloalkylalkyle, à alkyler un composé (Ia) par un agent d'alkylation $R_3$-X, X étant un halogène, ou à réduire un composé (Ic) par un hydrure de carbone ou d'aluminium, ou à réduire un composé de formule

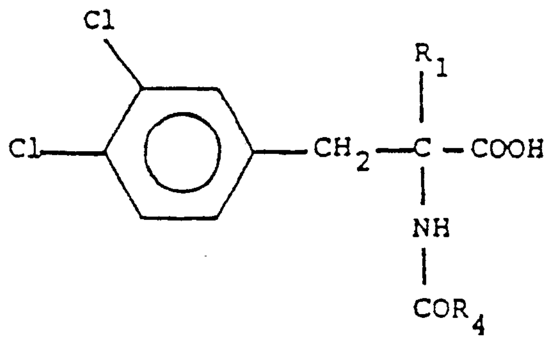

(IV)

par un hydrure de bore ou d'aluminium, et

pour préparer les composés (Ie), dans lesquels $R_2$ est alkyle et $R_3$ est identique à $R_2$, à faire réagir un composé (Ia) sur un aldéhyde de formule $R_4CHO$ en présence d'acide formique, et

pour préparer les composés (Ie) dans lesquels $R_2$ est alkyle et $R_3$ est alkyle, alkényle, phénylalkyle ou cycloalkylalkyle, à faire réagir un composé (Id) sur un aldéhyde de formule $R_4CHO$ en présence d'acide formique et, pour préparer les sels d'addition avec des acides, à faire réagir les composés (I) sur un acide.

2. Procédé suivant la revendication 1, caractérisé en ce que $R_1$ est méthyle.

3. Procédé suivant la revendication 1 ou 2, caractérisé en ce que $R_2$ est hydrogène ou méthyle.

4. Procédé suivant la revendication 1, 2 ou 3, caractérisé en ce que $R_3$ est hydrogène ou méthyle.

5. Procédé suivant la revendication 1, caractérisé en ce que l'aminoalcool est le 2-méthyl-2-amino-3-(3,4-dichlorophényl)propanol ou ses sels.

6. Procédé suivant la revendication 1, caractérisé en ce que l'aminoalcool est le 2-méthyl-2-méthylamino-3-(3,4-dichlorophényl)propanol ou ses sels.

7. Procédé suivant la revendication 1, caractérisé en ce que l'aminoalcool est le 2-méthyl-2-éthylamino-3-(3,4-dichlorophényl)propanol ou ses sels,

8. Procédé suivant la revendication 1, caractérisé en ce que l'aminoalcool est le 2-méthyl-2-allylamino-3-(3,4-dichlorophényl)propanol ou ses sels,

9. Procédé suivant la revendication 1, caractérisé en ce que l'aminoalcool est le 2-méthyl-2-cyclopropaneméthylamino-3-(3,4-dichlorophényl)propanol et ses sels,

10. Procédé suivant la revendication 1, caractérisé en ce que l'aminoalcool est le 2-méthyl-2-(2,2-diméthyl)propylamino-3-(3,4-dichlorophényl)propanol et ses sels,

11. Procédé suivant la revendication 1, caractérisé en ce que l'aminoalcool est le 2-méthyl-2-(2-phényl)éthylamino-3-(3,4-dichlorophényl)propanol et ses sels,

12. Procédé suivant la revendication 1, caractérisé en ce que l'aminoalcool est le 2-méthyl-2-(N-méthyl, N-éthyl)amino-3-(3,4-dichlorophényl)propanol et ses sels,

13. Procédé suivant la revendication 1, caractérisé en ce que l'aminoalcool est le 2-méthyl-2-[N-méthyl, N-(2-phényl)éthyl]amino-3-(3,4-dichlorophényl)propanol et ses sels,

14. Procédé suivant la revendication 1, caractérisé en ce que l'aminoalcool est le 2-isopropyl-2-amino-3-(3,4-dichlorophényl)propanol et ses sels,

15. Procédé suivant la revendication 1, caractérisé en ce que l'aminoalcool est le 2-méthyl-2-(N-méthyl, N-allyl)amino-3-(3,4-dichlorophényl)propanol et ses sels.

16. Procédé de préparation d'une composition pharmaceutique ayant notamment des activités sur le système nerveux central et analgésique, caractérisé, en ce qu'il consiste à mélanger un aminoalcool préparé suivant l'une des revendications précédentes à un excipient pharmaceutique.

17. L'utilisation d'un aminoalcool préparé suivant l'une des revendications 1 à 15 pour l'obtention d'un médicament agissant sur le système nerveux central et ayant en outre une activité analgésique.

**Revendications** (pour l'Etat contractant GR)

1. Aminoalcools de formule

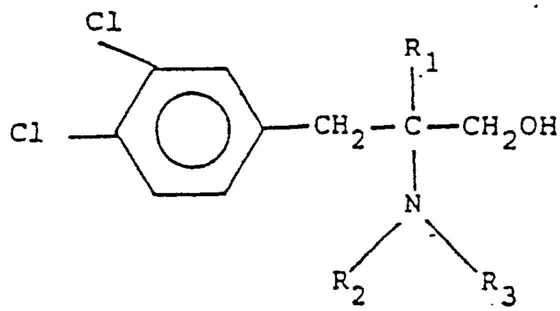

(I)

dans laquelle :

$R_1$ est alkyle inférieur,

$R_2$ est H ou alkyle inférieur,

$R_3$ est H, alkyle inférieur, alkényle inférieur, phénylalkyle inférieur ou cycloalkylalkyle inférieur ayant de 3 à 6 atomes de carbone dans le cycle, ou, quand $R_2$ est H, alcanoyle inférieur, phénylalcanoyle inférieur ou cycloalkylcarbonyle ayant de 3 à 6 atomes de carbone dans le cycle, ou $R_2$ et $R_3$ forment, ensemble avec l'atome d'azote auquel ils sont reliés, un hétérocycle saturé de cinq à sept chaînons pouvant comprendre, comme second hétéroatome non relié directement à l'atome d'azote, un oxygène, un soufre ou un azote, ce dernier hétéroatome d'azote pouvant porter un substituant alkyle en $C_1$ à $C_4$, et leurs sels d'addition avec des acides.

2. Aminoalcools suivant la revendication 1, caractérisés en ce que $R_1$ est méthyle.

3. Aminoalcools suivant la revendication 1 ou 2, caractérisés en ce que $R_2$ est hydrogène ou méthyle.

4. Aminoalcools suivant la revendication 1, 2 ou 3, caractérisés en ce que $R_3$ est hydrogène ou méthyle.

5. Le 2-méthyl-2-amino-3-(3,4-dichlorophényl)propanol et ses sels,

6. Le 2-méthyl-2-méthylamino-3-(3,4-dichlorophényl)propanol et ses sels,

7. Le 2-méthyl-2-éthylamino-3-(3,4-dichlorophényl)propanol et ses sels,

8. Le 2-méthyl-2-allylamino-3-(3,4-dichlorophényl)propanol et ses sels,

9. Le 2-méthyl-2-cyclopropaneméthylamino-3-(3,4-dichlorophényl)propanol et ses sels,

10. Le 2-méthyl-2-(2,2-diméthyl-propylamino-3-(3,4-dichlorophényl)propanol et ses sels,

11. Le 2-méthyl-2-(2-phényl)éthylamino-3-(3,4-dichlorophényl)propanol et ses sels,

12. Le 2-méthyl-2-(N-méthyl, N-éthyl)amino-3-(3,4-dichlorophényl)propanol et ses sels,

13. Le 2-méthyl-2-[N-méthyl, N-(2-phényl)éthyl]amino-3-(3,4-dichlorophényl)propanol et ses sels,

14. Le 2-méthyl-2-(N-méthyl, N-allyl) amino-3-(3,4-dichlorophényl) propanol et ses sels,

15. Procédé de préparation des composés suivant la revendication 1, caractérisé en ce qu'il consiste :

à réduire un acide de formule

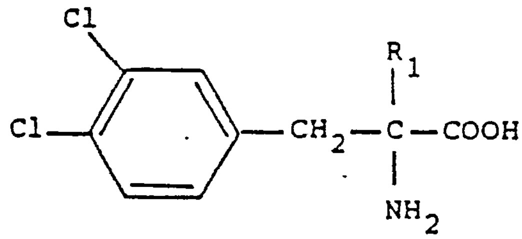

(II)

par un hydrure de bore ou d'aluminium pour obtenir les composés (Ia) dans lesquels $R_2$ et $R_3$ sont l'hydrogène, et

pour obtenir les composés (Ib), dans lesquels $NR_2R_3$ forment un hétérocyle, à effectuer une cyclisation par réaction d'un composé (Ia) sur un réactif de formule

$$X—(CH_2)_m—R_5—(CH_2)_n—X'$$

dans laquelle X et X', semblables ou différents, sont halogènes, $R_5$ est une liaison simple de valence et m et n sont des nombres entiers allant de 1 à 3, avec m + n supérieur ou égal à 4 et inférieur ou égal à 6, ou $R_5$ est un oxygène, un soufre ou $NR_6$, $R_6$ étant H ou alkyle en $C_1$ à $C_4$ et m et n sont des nombres entiers allant de 1 à 3 avec m + n supérieur ou égal à 3 et inférieur ou égal à 5, et

pour préparer les composés (Ic) dans lesquels $R_3$ est alkanoyle, phénylalkanoyle ou cycloalkylcarbonyle, à acyler un composé (Ia) par un agent d'acylation de formule $R_4$-COX dans laquelle X est un halogène et $R_4$ est l'homologue immédiatement inférieur de $R_3$, et

pour préparer les composés Id, dans lesquels $R_2$ est H et $R_3$ est alkyle, alkényle, phénylalkyle, ou cycloalkylalkyle, à alkyler un composé (Ia) par un agent d'alkylation $R_3$-X, X étant un halogène, ou à réduire un composé (Ic) par un hydrure de carbone ou d'aluminium, ou à réduire un composé de formule

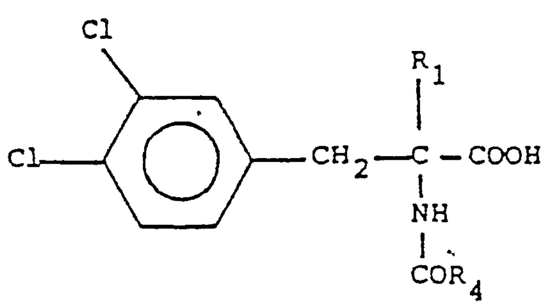

(IV)

par un hydrure de bore ou d'aluminium, et

pour préparer les composés (Ie), dans lesquels $R_2$ est alkyle et $R_3$ est identique à $R_2$, à faire réagir un composé (Ia) sur un aldéhyde de formule $R_4CHO$ en présence d'acide formique, et

pour préparer les composés (Ie) dans lesquels $R_2$ est alkyle et $R_3$ est alkyle, alkényle, phénylalkyle ou cycloalkylalkyle, à faire réagir un composé (Id) sur un aldéhyde de formule $R_4CHO$ en présence d'acide formique et, pour préparer les sels d'addition avec des acides, à faire réagir les composés (I) sur un acide.

16. Procédé de préparation d'une composition pharmaceutique ayant notamment des activités sur le système nerveux central et analgésique, caractérisé en ce qu'il consiste à mélanger un aminoalcool suivant l'une des revendications 1-14 à un excipient pharmaceutique.

17. L'utilisation d'un aminoalcool défini aux revendications 1 à 14 pour l'obtention d'un médicament agissant sur le système nerveux central et ayant, en outre, une activité analgésique.

**Claims** (for the Contracting States : BE, CH, DE, FR, GB, IT, LI, LU, NL, SE)

1. Aminoalcohols of formula

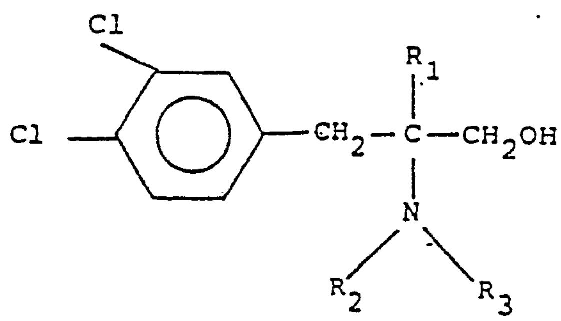

(I)

in which

$R_1$ is lower alkyl and

$R_2$ is H or lower alkyl,

$R_3$ is H, lower alkyl, lower alkenyl, lower phenylalkyl or lower cycloalkylalkyl with 3 to 6 carbon atoms in the cycle or, when $R_2$ is H, lower alkanoyl, lower phenylalkanoyl or cycloalkylcarbonyl having 3 to 6 carbon atoms in the cycle, or $R_2$ and $R_3$ form together with the nitrogen atom to which they are linked, a heterocycle saturated with 5 to 7 chain links which can have, as the second heteroatom not directly connected to the nitrogen atom, an oxygen, a sulphur or a nitrogen, the latter nitrogen heteroatom possibly carrying an alkyl substituent in $C_1$ to $C_4$, and their acid addition salts.

2. Aminoalcohols according to claim 1, characterized in that $R_1$ is methyl.

3. Aminoalcohols according to claims 1 or 2, characterized in that $R_2$ is hydrogen or methyl.

4. Aminoalcohols according to claims 1, 2 or 3, characterized in that $R_3$ is hydrogen or methyl.

5. 2-methyl-2-amino-3-(3,4-dichlorophenyl)-propanol and its salts.

6. 2-methyl-2-methylamino-3-(3,4-dichlorophenyl)propanol and its salts.

7. 2-methyl-2-ethylamino-3-(3,4-dichlorophenyl)-propanol and its salts.

8. 2-methyl-2-allylamino-3-(3,4-dichlorophenyl)-propanol and its salts.

9. 2-methyl-2-cyclopropanmethylamino-3-(3,4-dichlorophenyl)propanol and its salts.

10. 2-methyl-2-(2,2-dimethyl-propylamino-3-(3,4-dichlorophenyl)propanol and its salts.

11. 2-methyl-2-(2-phenyl)ethylamino-3-(3,4-dichlorophenyl)propanol and its salts.

12. 2-methyl-2-(N-methyl, N-ethyl)amino-3-(3,4-dichlorophenyl)propanol and its salts.

13. 2-methyl-2-[N-methyl, N-(2-phenyl)ethyl]amino-3-(3,4-dichlorophenyl)propanol and its salts.

14. 2-methyl-2-(N-methyl, N-allyl)amino-3-(3,4-dichlorophenyl)propanol and its salts.

15. Process for the preparation of aminoalcohols as claimed in claim 1, characterized in that it comprises :

reducing an acid of formula

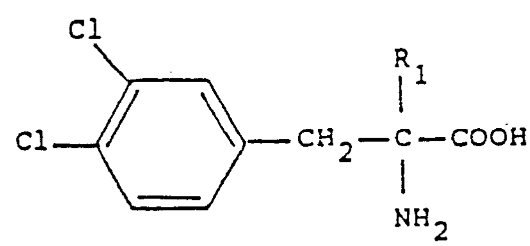

(II)

by a boron or aluminium hydride to obtain compounds (Ia), in which $R_2$ and $R_3$ are hydrogen and

for obtaining compounds (Ib), in which $NR_2R_3$ form a heterocycle performing a cyclization by reacting a compound (Ia) with a reagent of formula

$$X—(CH_2)_m—R_5—(CH_2)_n—X'$$

in which X and X', which can be the same or different, are halogen, $R_5$ is a single valence bond and m and n are integers between 1 and 3, with m + n equal to or greater than 4 and equal to or less than 6, or $R_5$ is an oxygen, a sulphur or $NR_6$, $R_6$ being H or alkyl in $C_1$ to $C_4$ and m and n are integers between 1 and 3 with m + n equal to or higher than 3 and equal to or lower than 5 and

for preparing compounds (Ic), in which $R_3$ is alkanoyl, phenylalkanoyl or cycloalkylcarbonyl, acylating a compound (Ia) with an acylating agent of formula $R_4$-COX, in which X is a halogen and $R_4$ is the homolog immediately below $R_3$ and

for preparing compounds (Id), in which $R_2$ is H and $R_3$ is alkyl, alkenyl, phenylalkyl or cycloalkylalkyl, alkylating a compound (Ia) using an alkylating agent $R_3$-X, X being a halogen, or reducing a compound (Ic) by a boron or aluminium hydride, or reducing a compound of formula

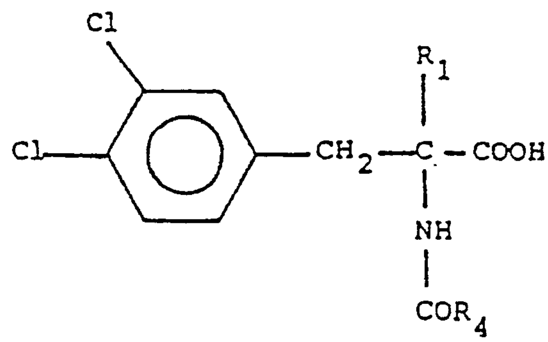

(IV)

by a boron or aluminium hydride and

for preparing compounds (Ie), in which $R_2$ is alkyl and $R_3$ identical to $R_2$, reacting a compound (Ia) with an aldehyde of formula $R_4$CHO in the presence of formic acid and

for preparing compounds (Ie) in which $R_2$ is alkyl and $R_3$ is alkyl, alkenyl, phenylalkyl or cycloalkylalkyl reacting a compound (Id) with an aldehyde of formula $R_4$CHO in the presence of formic acid and, for preparing the acid addition salts, reacting the compounds (I) with an acid.

16. Analgesic medicament having an activity on the central nervous system comprising as active ingredient an aminoalcohol as claimed in claims 1 to 14.

**Claims** (for the Contracting States : AT, ES)

1. Process for the preparation of aminoalcohols of formula

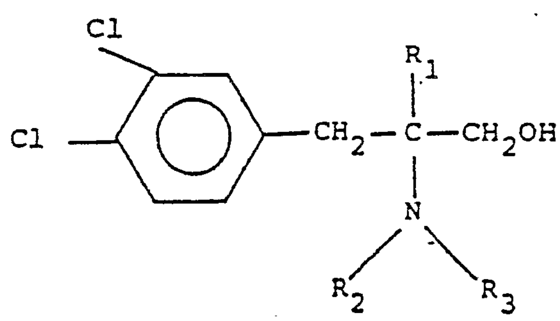

(I).

in which

$R_1$ is lower alkyl and

$R_2$ is H or lower alkyl,

$R_3$ is H, lower alkyl, lower alkenyl, lower phenylalkyl or lower cycloalkylalkyl with 3 to 6 carbon atoms in the cycle, or when $R_2$ is H, lower alkanoyl, lower phenylalkanoyl or cycloalkylcarbonyl having 3 to 6 carbon atoms in the cycle, or $R_2$ and $R_3$ form together with the nitrogen atom to which they are linked, a heterocycle saturated with 5 to 7 chain links and which can comprise, as the second heteroatom not directly linked with the nitrogen atom, an oxygen, a sulphur or a nitrogen, whereby said latter nitrogen heteroatom can carry an alkyl substituent in $C_1$ to $C_4$ and their acid addition salts, characterized in that it comprises :

reducing an acid of formula

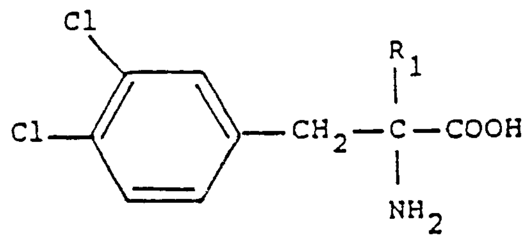

(II)

by a boron or aluminium hydride to obtain compounds (Ia), in which $R_2$ and $R_3$ are hydrogen and

for obtaining compounds (Ib), in which $NR_2R_3$ form a heterocycle performing a cyclization by reacting a compound (Ia) with a reagent of formula

$$X—(CH_2)_m—R_5—(CH_2)_n—X'$$

in which X and X', which can be the same or different, are halogen, $R_5$ is a single valence bond and m and n are integers between 1 and 3, with m + n equal to or greater than 4 and equal to or less than 6, or $R_5$ is an oxygen, a sulphur or $NR_6$, $R_6$ being H or alkyl in $C_1$ to $C_4$ and m and n are integers between 1 and 3 with m + n equal to or higher than 3 and equal to or lower than 5 and

for preparing compounds (Ic), in which $R_3$ is alkanoyl, phenylalkanoyl or cycloalkylcarbonyl, acylating a compound (Ia) with an acylating agent of formula $R_4$-COX, in which X is a halogen and $R_4$ is the homolog immediately below $R_3$ and

for preparing compounds (Id), in which $R_2$ is H and $R_3$ is alkyl, alkenyl, phenylalkyl or cycloalkylalkyl, alkylating a compound (Ia) using an alkylating agent $R_3$-X, X being a halogen, or reducing a compound (Ic) by a boron or aluminium hydride, or reducing a compound of formula

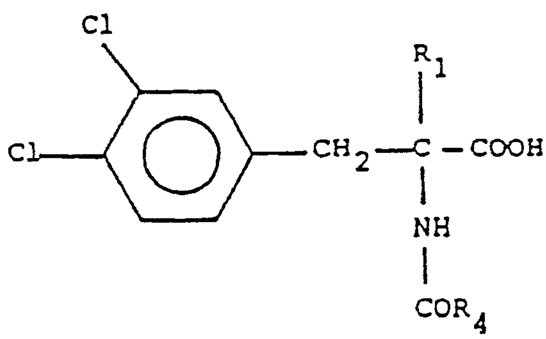

(IV)

by a boron or aluminium hydride and

for preparing compounds (Ie), in which $R_2$ is alkyl and $R_3$ identical to $R_2$, reacting a compound (Ia) with an aldehyde of formula $R_4CHO$ in the presence of formic acid and

for preparing compounds (Ie) in which $R_2$ is alkyl and $R_3$ is alkyl, alkenyl, phenylalkyl or cycloalkylalkyl, reacting a compound (Id) with an aldehyde of formula $R_4CHO$ in the presence of formic acid and, for preparing the acid addition salts, reacting the compounds (I) with an acid.

2. Process according to claim 1, characterized in that $R_1$ is methyl.

3. Process according to claims 1 or 2, characterized in that $R_2$ is hydrogen or methyl.

4. Process according to claims 1, 2 or 3, characterized in that $R_3$ is hydrogen or methyl.

5. Process according to claim 1, characterized in that the aminoalcohol is 2-methyl-2-amino-3-(3,4-dichlorophenyl)propanol or its salts.

6. Process according to claim 1, characterized in that the aminoalcohol is 2-methyl-2-methylamino-3-(3,4-dichlorophenyl)propanol or its salts.

7. Process according to claim 1, characterized in that the aminoalcohol is 2-methyl-2-ethylamino-3-(3,4-dichlorophenyl)-propanol or its salts.

8. Process according to claim 1, characterized in that the aminoalcohol is 2-methyl-2-allylamino-3-(3,4-dichlorophenyl)-propanol or its salts.

9. Process according to claim 1, characterized in that the aminoalcohol is 2-methyl-2-cyclopropanmethylamino-3-(3,4-dichlorophenyl)propanol and its salts.

10. Process according to claim 1, characterized in that the aminoalcohol is 2-methyl-2-(2,2-dimethylpropylamino-3-(3,4-dichlorophenyl)propanol and its salts.

11. Process according to claim 1, characterized in that the aminoalcohol is 2-methyl-2-(2-phenyl)ethylamino-3-(3,4-dichlorophenyl)propanol and its salts.

12. Process according to claim 1, characterized in that the aminoalcohol is 2-methyl-2-(N-methyl, N-ethyl)amino-3-(3,4-dichlorophenyl)propanol and its salts.

13. Process according to claim 1, characterized in that the aminoalcohol is 2-methyl-2-[N-methyl, N-(2-phenyl)ethyl]amino-3-(3,4-dichlorophenyl)propanol and its salts.

14. Process according to claim 1, characterized in that the aminoalcohol is 2-isopropyl-2-amino-3-(3,4-dichlorophenyl)propanol and its salts.

15. Process according to claim 1, characterized in that the aminoalcohol is 2-methyl-2-(N-methyl, N-allyl)amino-3-(3,4-dichlorophenyl)propanol and its salts.

16. Process for preparing a pharmaceutical composition having in particular activity on the central nervous system and analgesic activity, characterized in that it comprises mixing an aminoalcohol prepared according to one of the preceeding claims with a pharmaceutical carrier.

17. The use of an aminoalcohol prepared according to one of claims 1 to 15 for preparing a medicament which is active on the central nervous system and which has further analgesic activity.

**Claims** (for the Contracting State GR)

1. Aminoalcohols of formula

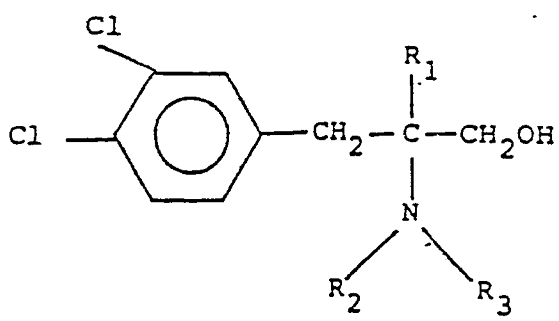

(I)

in which

$R_1$ is lower alkyl and

$R_2$ is H or lower alkyl,

$R_3$ is H, lower alkyl, lower alkenyl, lower phenylalkyl or lower cycloalkylalkyl with 3 to 6 carbon atoms in the cycle or, when $R_2$ is H, lower alkanoyl, lower phenylalkanoyl or cycloalkylcarbonyl having 3 to 6 carbon atoms in the cycle, or $R_2$ and $R_3$ form together with the nitrogen atom to which they are linked, a heterocycle saturated with 5 to 7 chain links which can have, as the second heteroatom not directly connected to the nitrogen atom, an oxygen, a sulphur or a nitrogen, the latter nitrogen heteroatom possibly carrying an alkyl substituent in $C_1$ to $C_4$, and their acid addition salts.

2. Aminoalcohols according to claim 1, characterized in that $R_1$ is methyl.

3. Aminoalcohols according to claims 1 or 2, characterized in that $R_2$ is hydrogen or methyl.

4. Aminoalcohols according to claims 1, 2 or 3, characterized in that $R_3$ is hydrogen or methyl.

5. 2-methyl-2-amino-3-(3,4-dichlorophenyl)-propanol and its salts.

6. 2-methyl-2-methylamino-3-(3,4-dichlorophenyl)propanol and its salts.

7. 2-methyl-2-ethylamino-3-(3,4-dichlorophenyl)-propanol and its salts.

8. 2-methyl-2-allylamino-3-(3,4-dichlorophenyl)-propanol and its salts.

9. 2-methyl-2-cyclopropanmethylamino-3-(3,4-dichlorophenyl)propanol and its salts.

10. 2-methyl-2-(2,2-dimethyl-propylamino-3-(3,4-dichlorophenyl)propanol and its salts.

11. 2-methyl-2-(2-phenyl)ethylamino-3-(3,4-dichlorophenyl)propanol and its salts.

12. 2-methyl-2-(N-methyl, N-ethyl)amino-3-(3,4-dichlorophenyl)propanol and its salts.

13. 2-methyl-2-[N-methyl, N-(2-phenyl)ethyl]amino-3-(3,4-dichlorophenyl)propanol and its salts.

14. 2-methyl-2-(N-methyl, N-allyl)amino-3-(3,4-dichlorophenyl)propanol and its salts.

15. Process for the preparation of aminoalcohols as claimed in claim 1, characterized in that it comprises :

reducing an acid of formula

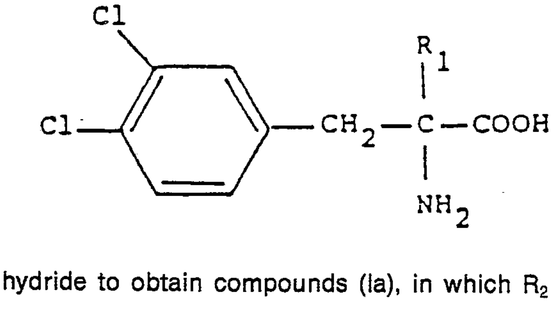

(II)

by a boron or aluminium hydride to obtain compounds (Ia), in which $R_2$ and $R_3$ are hydrogen and

for obtaining compounds (Ib), in which $NR_2R_3$ form a heterocycle performing a cyclization by reacting a compound (Ia) with a reagent of formula

$$X—(CH_2)_m—R_5—(CH_2)_n—X'$$

in which X and X', which can be the same or different, are halogen, $R_5$ is a single valence bond and m and n are integers between 1 and 3, with m + n equal to or greater than 4 and equal to or less than 6, or $R_5$ is an oxygen, a sulphur or $NR_6$, $R_6$ being H or alkyl in $C_1$ to $C_4$ and m and n are integers between 1 and 3 with m + n equal to or higher than 3 and equal to or lower than 5 and

for preparing compounds (Ic), in which $R_3$ is alkanoyl, phenylalkanoyl or cycloalkylcarbonyl, acylating a compound (Ia) with an acylating agent of formula $R_4$-COX, in which X is a halogen and $R_4$ is the homolog immediately below $R_3$ and

for preparing compounds (Id), in which $R_2$ is H and $R_3$ is alkyl, alkenyl, phenylalkyl or cycloalkylalkyl, alkylating a compound (Ia) using an alkylating agent $R_3$-X, X being a halogen, or reducing a compound (Ic) by a boron or aluminium hydride, or reducing a compound of formula

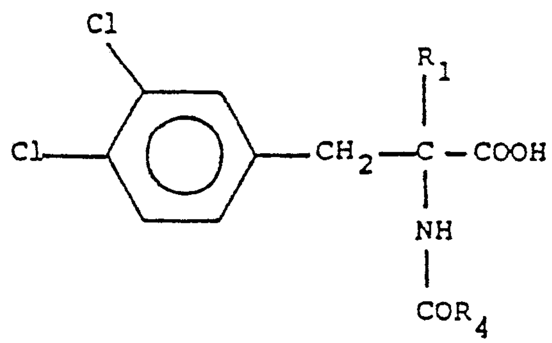

(IV)

by a boron or aluminium hydride and

for preparing compounds (Ie), in which $R_2$ is alkyl and $R_3$ identical to $R_2$, reacting a compound (Ia) with an aldehyde of formula $R_4CHO$ in the presence of formic acid and

for preparing compounds (Ie) in which $R_2$ is alkyl and $R_3$ is alkyl, alkenyl, phenylalkyl or cycloalkylalkyl reacting a compound (Id) with an aldehyde of formula $R_4CHO$ in the presence of formic acid and, for preparing the acid addition salts, reacting the compounds (I) with an acid.

16. Process for preparing a pharmaceutical composition having in particular activity on the central nervous system and analgesic activity, characterized in that it comprises mixing an aminoalcohol as claimed in claims 1-14 with a pharmaceutical carrier.

17. The use of an aminoalcohol defined in claims 1-15 for preparing a medicament which is active on the central nervous system and which has further analgesic activity.

**Patentansprüche** (für die Vertragsstaaten : BE, CH, DE, FR, GB, IT, LI, LU, NL, SE)

1. Aminoalkohole der Formel

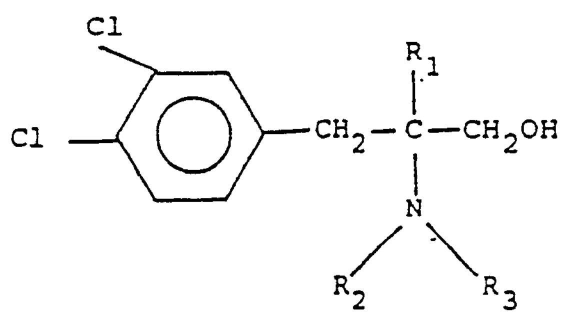

(I)

worin bedeuten :

$R_1$ Niedrigalkyl,

$R_2$ Wasserstoff oder Niedrigalkyl,

$R_3$ Wasserstoff, Niedrigalkyl, Niedrigalkenyl, Phenylniedrigalkyl oder Cycloniedrigalkylalkyl mit 3 bis 6 Kohlenstoffatomen im Ring, oder, wenn $R_2$ Wasserstoff ist, Niedrigalkanoyl, Phenylniedrigalkanoyl oder Cycloalkylcarbonyl mit 3 bis 6 Kohlenstoffatomen im Ring, oder $R_2$ und $R_3$ bilden zusammengenommen mit dem Kohlenstoffatom, an das sie gebunden sind, einen gesättigten 5- bis 7-gliedrigen Heterocyclus, der als zweites Heteroatom, das nicht direkt mit dem Stickstoffatom verbunden ist, ein Sauerstoffatom,

ein Schwefelatom oder ein Stickstoffatom enthalten kann, wobei das letztere Heterostickstoffatom einen $C_1$ bis $C_4$ Alkylsubstituenten tragen kann und ihre Säureadditionssalze.

2. Aminoalkohole nach Anspruch 1, dadurch gekennzeichnet, daß $R_1$ Methyl ist.

3. Aminoalkohole nach Anspruch 1 oder 2, dadurch gekennzeichnet, daß $R_2$ Wasserstoff oder Methyl ist.

4. Aminoalkohole nach Anspruch 1, 2 oder 3, dadurch gekennzeichnet, daß $R_3$ Wasserstoff oder Methyl ist.

5. 2-Methyl-2-amino-3-(3,4-dichlorphenyl)propanol und seine Salze.

6. 2-Methyl-2-methylamino-3-(3,4-dichlorphenyl)propanol und seine Salze.

7. 2-Methyl-2-ethylamino-3-(3,4-dichlorphenyl)propanol und seine Salze.

8. 2-Methyl-2-allylamino-3-(3,4-dichlorphenyl)propanol und seine Salze.

9. 2-Methyl-2-cyclopropanmethylamino-3-(3,4-dichlorphenyl)propanol und seine Salze.

10. 2-Methyl-2-(2,2-dimethyl-propylamino-3-(3,4-dichlorphenyl)propanol und seine Salze.

11. 2-Methyl-2-(2-phenyl)ethylamino-3-(3,4-dichlorphenyl)propanol und seine Salze.

12. 2-Methyl-2-(N-methyl, N-ethyl)amino-3-(3,4-dichlorphenyl)propanol und seine Salze.

13. 2-Methyl-2-[N-methyl, N-(2-phenyl)ethyl]amino-3-(3,4-dichlorphenyl)propanol und seine Salze.

14. 2-Methyl-2-(N-methyl, N-allyl)-amino-3-(3,4-dichlorphenyl)propanol und seine Salze.

15. Verfahren zur Herstellung der Verbindungen nach Anspruch 1, dadurch gekennzeichnet, daß man :

eine Säure der Formel

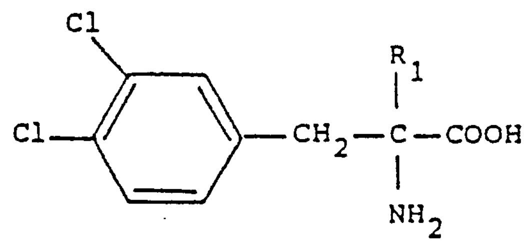

(II)

mit Borhydrid oder Aluminiumhydrid reduziert um die Verbindungen (Ia) zu erhalten, worin $R_2$ und $R_3$ Wasserstoff sind, und

um die Verbindungen (Ib) zu erhalten, worin $NR_2R_3$ einen Heterocyclus bilden, eine Cyclisierungsreaktion durch Umsetzen einer Verbindung (Ia) mit einem Reagenz der Formel

$$X—(CH_2)_m—R_5—(CH_2)_n—X'$$

durchführt, worin X und X', die gleich oder verschieden sind, ein Halogen bedeuten, $R_5$ ist eine Einfachbindung und m und n sind ganze Zahlen von 1 bis 3, wobei m + n größer oder gleich 4 und kleiner oder gleich 6 ist, oder $R_5$ ist ein Sauerstoffatom, ein Schwefelatom oder $NR_6$, worin $R_6$ ein Wasserstoffatom oder $C_1$ bis $C_4$ Alkyl ist und m + n ganze Zahlen von 1 bis 3 sind mit m + n größer oder gleich 3 und kleiner oder gleich 5, und

zur Herstellung der verbindungen (Ic), worin $R_3$ Alkanoyl, Phenylalkanoyl oder Cycloalkylcarbonyl bedeutet, eine Verbindung (Ia) mit einem Acylierungsreagenz der Formel $R_4$-COX, worin X ein Halogenatom ist und $R_4$ das unmittelbar niedrigere Homologe von $R_3$, acyliert, und

zur Herstellung der Verbindungen Id, worin $R_2$ Wasserstoff und $R_3$ Alkyl, Alkenyl, Phenylalkyl oder Cycloalkylalkyl ist eine Verbindung (Ia) mit einem Alkylierungsreagenz $R_3$-X alkyliert, worin X ein Halogenatom ist oder eine Verbindung (Ic) mit einem Kohlenstoff- oder Aluminiumhydrid reduziert oder eine Verbindung der Formel

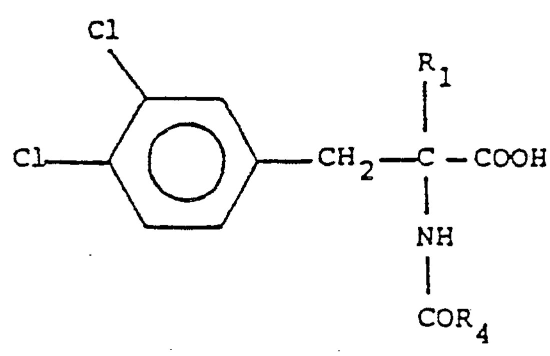

(IV)

mit Borhydrid oder Aluminiumhydrid reduziert, und

zur Herstellung der Verbindungen (Ie), worin $R_2$ Alkyl bedeutet und $R_3$ mit $R_2$ identisch ist, eine

Verbindung (Ia) mit einem Aldehyd der Formel $R_4CHO$ in Gegenwart von Ameisensäure umsetzt, und

zur Herstellung der Verbindungen (Ie), worin $R_2$ Alkyl ist und $R_3$ Alkyl, Alkenyl, Phenylalkyl oder Cycloalkylalkyl ist, eine Verbindung (Id) mit einem Aldehyd der Formel $R_4CHO$ in Gegenwart von Ameisensäure umsetzt und zur Herstellung der Säureadditionssalze, die Verbindungen (I) mit einer Säure umsetzt.

16. Analgetisches Medikament mit einer Aktivität auf das Zentrale Nervensystem, dadurch gekennzeichnet, daß es als aktiven Bestandteil einen Aminoalkohol nach einem der Ansprüche 1 bis 14 enthält.

**Patentansprüche** (für die Vertragsstaaten : AT, ES)

1. Verfahren zur Herstellung von Aminoalkoholen der Formel

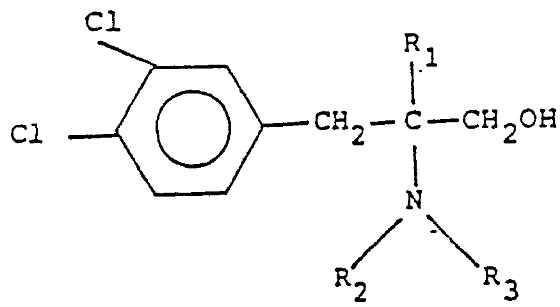

(I)

worin bedeuten :

$R_1$ Niedrigalkyl,

$R_2$ Wasserstoff oder Niedrigalkyl,

$R_3$ Wasserstoff, Niedrigalkyl, Niedrigalkenyl, Phenylniedrigalkyl oder Cycloniedrigalkylalkyl mit 3 bis 6 Kohlenstoffatomen im Ring, oder, wenn $R_2$ Wasserstoff ist, Niedrigalkanoyl, Phenylniedrigalkanoyl oder Cycloalkylcarbonyl mit 3 bis 6 Kohlenstoffatomen im Ring, oder $R_2$ und $R_3$ bilden zusammengenommen mit dem Kohlenstoffatom, an das sie gebunden sind, einen gesättigten 5- bis 7-gliedrigen Heterocyclus, der als zweites Heteroatom, das nicht direkt mit dem Stickstoffatom verbunden ist, ein Sauerstoffatom, ein Schwefelatom oder ein Stickstoffatom enthalten kann, wobei das letztere Heterostickstoffatom einen $C_1$ bis $C_4$ Alkylsubstituenten tragen kann und ihre Säureadditionssalze, dadurch gekennzeichnet, daß man :

eine Säure der Formel

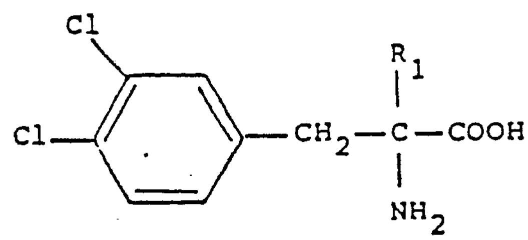

(II)

mit Borhydrid oder Aluminiumhydrid reduziert, um die Verbindungen (Ia) zu erhalten, worin $R_2$ und $R_3$ Wasserstoff sind, und

um die Verbindungen (Ib) zu erhalten, worin $NR_2R_3$ einen Heterocyclus bilden, eine Cyclisierungsreaktion durch Umsetzen einer Verbindung (Ia) mit einem Reagenz der Formel

$$X—(CH_2)_m—R_5—(CH_2)_n—X'$$

durchführt, worin X und X', die gleich oder verschieden sind, ein Halogen bedeuten, $R_5$ ist eine Einfachbindung und m und n sind ganze Zahlen von 1 bis 3, wobei m + n größer oder gleich 4 und kleiner oder gleich 6 ist, oder $R_5$ ist ein Sauerstoffatom, ein Schwefelatom oder $NR_6$, worin $R_6$ ein Wasserstoffatom oder $C_1$ bis $C_4$ Alkyl ist und m + n ganze Zahlen von 1 bis 3 sind mit m + n größer oder gleich 3 und kleiner oder gleich 5, und

zur Herstellung der verbindungen (Ic), worin $R_3$ Alkanoyl, Phenylalkanoyl oder Cycloalkylcarbonyl bedeutet, eine Verbindung (Ia) mit einem Acylierungsreagenz der Formel $R_4$-COX, worin X ein Halogenatom ist und $R_4$ das unmittelbar niedrigere Homologe von $R_3$, acyliert, und

zur Herstellung der Verbindungen Id, worin $R_2$ Wasserstoff und $R_3$ Alkyl, Alkenyl, Phenylalkyl oder Cycloalkylalkyl ist eine Verbindung (Ia) mit einem Alkylierungsreagenz $R_3$-X alkyliert, worin X ein Halogenatom ist oder eine Verbindung (Ic) mit einem Kohlenstoff- oder Aluminiumhydrid reduziert oder eine Verbindung der Formel

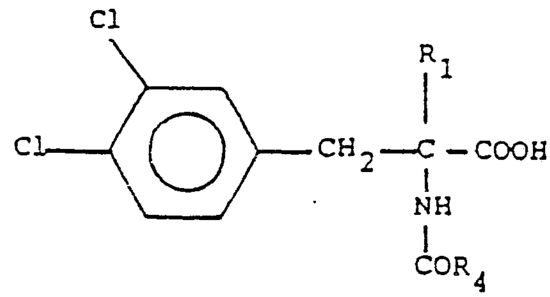

(IV)

mit Borhydrid oder Aluminiumhydrid reduziert, und

zur Herstellung der Verbindungen (Ie), worin $R_2$ Alkyl bedeutet und $R_3$ mit $R_2$ identisch ist, eine Verbindung (Ia) mit einem Aldehyd der Formel $R_4CHO$ in Gegenwart von Ameisensäure umsetzt, und

zur Herstellung der Verbindungen (Ie), worin $R_2$ Alkyl ist und $R_3$ Alkyl, Alkenyl, Phenylalkyl oder Cycloalkylalkyl ist, eine Verbindung (Id) mit einem Aldehyd der Formel $R_4CHO$ in Gegenwart von Ameisensäure umsetzt und zur Herstellung der Säureadditionssalze, die Verbindungen (I) mit einer Säure umsetzt.

2. Verfahren nach Anspruch 1, dadurch gekennzeichnet, daß $R_1$ Methyl ist.

3. Verfahren nach Anspruch 1 oder 2, dadurch gekennzeichnet, daß $R_2$ Wasserstoff oder Methyl ist.

4. Verfahren nach Anspruch 1, 2 oder 3, dadurch gekennzeichnet, daß $R_3$ Wasserstoff oder Methyl ist.

5. Verfahren nach Anspruch 1, dadurch gekennzeichnet, daß der Aminoalkohol 2-Methyl-2-amino-3-(3,4-dichlorphenyl)propanol oder seine Salze ist.

6. Verfahren nach Anspruch 1, dadurch gekennzeichnet, daß der Aminoalkohol 2-Methyl-2-methylamino-3-(3,4-dichlorphenyl)propanol oder seine Salze ist.

7. Verfahren nach Anspruch 1, dadurch gekennzeichnet, daß der Aminoalkohol 2-Methyl-2-ethylamino-3-(3,4-dichlorphenyl)propanol oder seine Salze ist.

8. Verfahren nach Anspruch 1, dadurch gekennzeichnet, daß der Aminoalkohol 2-Methyl-2-allylamino-3-(3,4-dichlorphenyl)propanol oder seine Salze ist.

9. Verfahren nach Anspruch 1, dadurch gekennzeichnet, daß der Aminoalkohol 2-Methyl-2-cyclopropanmethylamino-3-(3,4-dichlorphenyl)propanol oder seine Salze ist.

10. Verfahren nach Anspruch 1, dadurch gekennzeichnet, daß der Aminoalkohol 2-Methyl-2-(2,2-dimethyl-propylamino-3-(3,4-dichlorphenyl)propanol oder seine Salze ist.

11. Verfahren nach Anspruch 1, dadurch gekennzeichnet, daß der Aminoalkohol 2-Methyl-2-(2-phenyl)ethylamino-3-(3,4-dichlorphenyl)propanol oder seine Salze ist.

12. Verfahren nach Anspruch 1, dadurch gekennzeichnet, daß der Aminoalkohol 2-Methyl-2-(N-methyl, N-ethyl)amino-3-(3,4-dichlorphenyl)propanol oder seine Salze ist.

13. Verfahren nach Anspruch 1, dadurch gekennzeichnet, daß der Aminoalkohol 2-Methyl-2-[N-methyl, N-(2-phenyl)ethyl]amino-3-(3,4-dichlorphenyl)propanol oder seine Salze ist.

14. Verfahren nach Anspruch 1, dadurch gekennzeichnet, daß der Aminoalkohol 2-Isopropyl-2-amino-3-(3,4-dichlorphenyl)propanol oder seine Salze ist.

15. Verfahren nach Anspruch 1, dadurch gekennzeichnet, daß der Aminoalkohol 2-Methyl-2-(N-methyl, N-allyl)-amino-3-(3,4-dichlorphenyl)-propanol oder seine Salze ist.

16. Verfahren zur Herstellung einer pharmazeutischen Zusammensetzung mit insbesondere einer Aktivität auf das Zentrale Nervensystem und einer analgetischen Aktivität, dadurch gekennzeichnet, daß man einen Aminoalkohol, hergestellt nach einem der vorhergehenden Ansprüche mit einem pharmazeutischen Trägermaterial vermischt.

17. Verwendung eines Aminoalkohols, hergestellt nach einem der Ansprüche 1 bis 15 zur Herstellung eines Medikaments mit Wirkung auf das Zentrale Nervensystem und mit einer weiteren analgetischen Aktivität.

**Patentansprüche** (für den Vertragsstaat GR)

1. Aminoalkohole der Formel

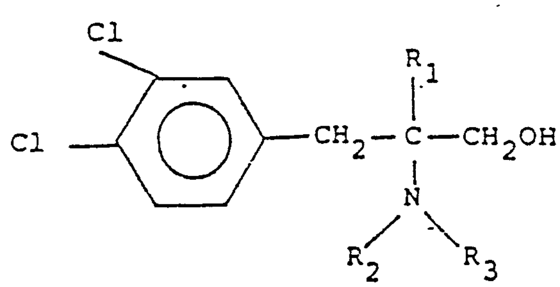

(I)

worin bedeuten :

$R_1$ Niedrigalkyl,

$R_2$ Wasserstoff oder Niedrigalkyl,

$R_3$ Wasserstoff, Niedrigalkyl, Niedrigalkenyl, Phenylniedrigalkyl oder Cycloniedrigalkylalkyl mit 3 bis 6 Kohlenstoffatomen im Ring, oder, wenn $R_2$ Wasserstoff ist, Niedrigalkanoyl, Phenylniedrigalkanoyl oder Cycloalkylcarbonyl mit 3 bis 6 Kohlenstoffatomen im Ring, oder $R_2$ und $R_3$ bilden zusammengenommen mit dem Kohlenstoffatom, an das sie gebunden sind, einen gesättigten 5- bis 7-gliedrigen Heterocyclus, der als zweites Heteroatom, das nicht direkt mit dem Stickstoffatom verbunden ist, ein Sauerstoffatom, ein Schwefelatom oder ein Stickstoffatom enthalten kann, wobei das letztere Heterostickstoffatom einen $C_1$ bis $C_4$ Alkylsubstituenten tragen kann und ihre Säureadditionssalze.

2. Aminoalkohole nach Anspruch 1, dadurch gekennzeichnet, daß $R_1$ Methyl ist.

3. Aminoalkohole nach Anspruch 1 oder 2, dadurch gekennzeichnet, daß $R_2$ Wasserstoff oder Methyl ist.

4. Aminoalkohole nach Anspruch 1, 2 oder 3, dadurch gekennzeichnet, daß $R_3$ Wasserstoff oder Methyl ist.

5. 2-Methyl-2-amino-3-(3,4-dichlorphenyl)propanol und seine Salze.

6. 2-Methyl-2-methylamino-3-(3,4-dichlorphenyl)propanol und seine Salze.

7. 2-Methyl-2-ethylamino-3(3,4-dichlorphenyl)propanol und seine Salze.

8. 2-Methyl-2-allylamino-3-(3,4-dichlorphenyl)propanol und seine Salze.

9. 2-Methyl-2-cyclopropanmethylamino-3-(3,4-dichlorphenyl)propanol und seine Salze.

10. 2-Methyl-2-(2,2-dimethyl-propylamino-3-(3,4-dichlorphenyl)propanol und seine Salze.

11. 2-Methyl-2-(2-phenyl)ethylamino-3-(3,4-dichlorphenyl)propanol und seine Salze.

12. 2-Methyl-2-(N-Methyl, N-ethyl)amino-3-(3,4-dichlorphenyl)propanol und seine Salze.

13. 2-Methyl-2-[N-methyl, N-(2-phenyl)ethyl]amino-3-(3,4-dichlorphenyl)propanol und seine Salze.

14. 2-Methyl-2-(N-methyl, N-allyl)-amino-3-(3,4-dichlorphenyl)propanol und seine Salze.

15. Verfahren zur Herstellung der Verbindungen nach Anspruch 1, dadurch gekennzeichnet, daß man :

eine Säure der Formel

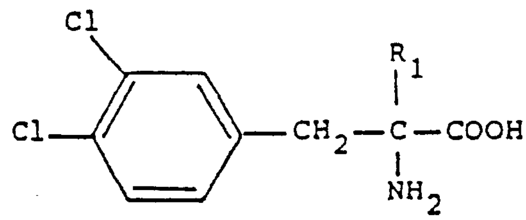

(II)

mit Borhydrid oder Aluminiumhydrid reduziert, um die Verbindungen (Ia) zu erhalten, worin $R_2$ und $R_3$ Wasserstoff sind, und

um die Verbindungen (Ib) zu erhalten, worin $NR_2R_3$ einen Heterocyclus bilden, eine Cyclisierungsreaktion durch Umsetzen einer Verbindung (Ia) mit einem Reagenz der Formel

$$X—(CH_2)_m—R_5—(CH_2)_n—X'$$

durchführt, worin X und X', die gleich oder verschieden sind, ein Halogen bedeuten, $R_5$ ist eine Einfachbindung und m und n sind ganze Zahlen von 1 bis 3, wobei m + n größer oder gleich 4 und kleiner oder gleich 6 ist, oder $R_5$ ist ein Sauerstoffatom, ein Schwefelatom oder $NR_6$, worin $R_6$ ein Wasserstoffatom oder $C_1$ bis $C_4$ Alkyl ist und m + n ganze Zahlen von 1 bis 3 sind mit m + n größer oder gleich 3 und kleiner oder gleich 5, und

zur Herstellung der Verbindungen (Ic), worin $R_3$ Alkanoyl, Phenylalkanoyl oder Cycloalkylcarbonyl bedeutet, eine Verbindung (Ia) mit einem Acylierungsreagenz der Formel $R_4$-COX, worin X ein Halogenatom ist und $R_4$ das unmittelbar niedrigere Homologe von $R_3$, acyliert, und

zur Herstellung der Verbindungen Id, worin $R_2$ Wasserstoff und $R_3$ Alkyl, Alkenyl, Phenylalkyl oder Cycloalkylalkyl ist eine Verbindung (Ia) mit einem Alkylierungsreagenz $R_3$-X alkyliert, worin X ein Halogenatom ist oder eine Verbindung (Ic) mit einem Kohlenstoff- oder Aluminiumhydrid reduziert oder eine Verbindung der Formel

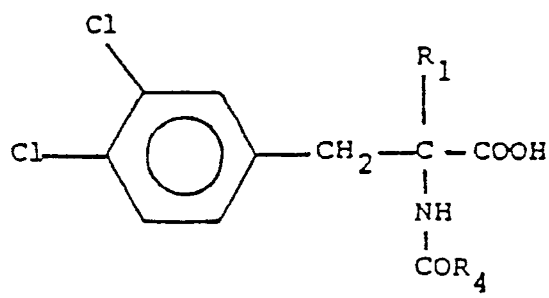

(IV)

mit Borhydrid oder Aluminiumhydrid reduziert, und

zur Herstellung der Verbindung (Ie), worin $R_2$ Alkyl bedeutet und $R_3$ mit $R_2$ identisch ist, eine Verbindung (Ia) mit einem Aldehyd der Formel $R_4CHO$ in Gegenwart von Ameisensäure umsetzt, und

zur Herstellung der Verbindungen (Ie), worin $R_2$ Alkyl ist und $R_3$ Alkyl, Alkenyl, Phenylalkyl oder Cycloalkylalkyl ist, eine Verbindung (Id) mit einem Aldehyd der Formel $R_4CHO$ in Gegenwart von Ameisensäure umsetzt und zur Herstellung der Säureadditionssalze, die Verbindungen (I) mit einer Säure umsetzt.

16. Verfahren zur Herstellung einer pharmazeutischen Zusammensetzung mit insbesondere Aktivitäten auf das Zentrale Nervensystem und analgetischer Aktivität, dadurch gekennzeichnet, daß man einen Aminoalkohol nach einem der Ansprüche 1 bis 14 mit einem pharmazeutischen Trägermaterial vermischt.

17. Verwendung eines Aminoalkohols, wie in den Ansprüchen 1 bis 14 definiert, zur Herstellung eines Medikaments mit Wirkung auf das Zentralnervensystem und weiterhin mit einer analgetischen Aktivität.